# FASCICULE DE BREVET EUROPEEN

(11) **EP 1 572 200 B1**
(45) Date de publication et mention de la délivrance du brevet: **17.08.2011**
(21) Numéro de dépôt: 03769560.8
(22) Date de dépôt: 03.09.2003
(51) Int. Cl.: A61K 31/416, C07D 231/56, A61P 25/00

(54) **DERIVES D'AMINOINDAZOLES ET LEUR UTILISATION COMME INHIBITEURS DE KINASES**
AMINOINDAZOLE DERIVATE ALS KINASE INHIBITOREN
AMINOINDAZOLE DERIVATIVES AND USE THEREOF AS KINASE INHIBITORS

(30) Priorité: 12.12.2002 FR 0215720; 04.02.2003 US 444630 P
(43) Date de publication de la demande: 14.09.2005
(73) Titulaire: Aventis Pharma S.A., 92160 Antony (FR)
(72) Inventeur: LESUISSE, Dominique, F-93100 MONTREUIL (FR); DUTRUC-ROSSET, Gilles, F-75012 PARIS (FR); HALLEY, Franck, F-92310 SEVRES (FR); BABIN, Didier, F-78180 MONTIGNY (FR); ROONEY, Thomas, F-91400 ORSAY (FR); TIRABOSCHI, Gilles, F-91230 MONTGERON (FR)
(74) Mandataire: Le Coupanec, Pascale A.M.P.
(86) Numéro de dépôt international: PCT/FR2003/002634
(87) Numéro de publication internationale: WO 2004/062662

(56) Documents cités:
- WO-A-02/022601
- WO-A-03/028720

## Description

La présente invention concerne l'utilisation de dérivés de formule (I): 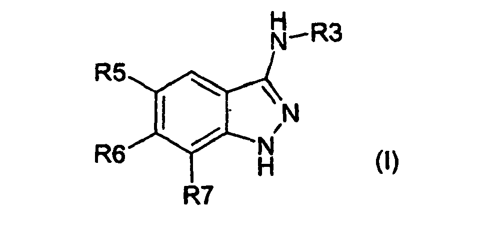 ou leurs sels pharmaceutiquement acceptables comme inhibiteur de kinase.

L'invention a pour objet l'utilisation des dérivés d'aminoindazoles de formule (I) et leurs sels pharmaceutiquement acceptables pour la préparation de compositions pharmaceutiques destinées à prévenir et traiter les maladies pouvant résulter d'une activité anormale de kinases comme par exemple celles impliquées dans les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer, les compositions pharmaceutiques contenant les nouveaux dérivés d'aminoindazoles et leurs sels pharmaceutiquement acceptables et les dérivés nouveaux d'aminoindazoles et leurs sels pharmaceutiquement acceptables.

La demande de brevet WO 02/074388 décrit des dérivés d'aminoindazole de type (a) activateurs des canaux potassium 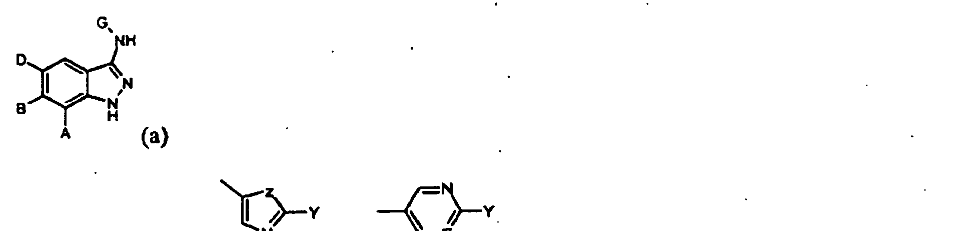 dans laquelle G est
Z est NX0, S, O
E est N, CX1
Y est halogène, X2, OX2
X0, X1, X2 sont halogène, alkyle ou un alkyle substitué
A, B, D sont hydrogène, halogène, alkyle substitué ou non, C(O)pR13, C(O)NR13R14, SO2NR13, R14, S(O)pR15, OR15, NR13R14
p est un entier de 0 à 2
R13, R14 est hydrogène, alkyle substitué ou non, cycloalkyle substitué ou non, hétéroaryle substitué ou non, hétérocycle substitué ou non, hétéroalkyle substitué ou non, hétéroaryle-hétéroalkyle substitué ou non, aryle-hétéroalkyle substitué ou non R15 alkyle substitué ou non, cycloalkyle substitué ou non, hétéroaryle substitué ou non, hétérocycle substitué ou non, hétéroalkyle substitué ou non, hétéroarylehétéroalkyle substitué ou non, arylehétéroalkyle substitué ou non.

La demande WO02/22601 décrit des dérivés de pyrazole utiles comme inhibiteurs de protéine kinase. La demande WO 03/028720 décrit des dérivés d'aminoindazole utiles comme inhibiteurs de protéine kinase.

La présente invention concerne des dérivés de formule (I) dans laquelle :
R3 est un radical (1-6C)alkyle, aryle, aryle(1-6C)alkyle, , hétéroaryle(1-6C)alkyle, hétérocycloalkyle, cycloalkyle, adamantyle, polycycloalkyles, alkényle, alkynyle, CONR1R2, CSNR1R2, CSNR1R2, COOR1, SO2R1, C(=NH)R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, hétérocycle, formyle, trifluorométhyle, trifluorométhylsulfanyle, trifluorométhoxy, (1-6C)alkyle ;
R5, R6, sont indépendamment l'un de l'autre choisis parmi les radicaux suivant halogène, CN, NO2, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle, adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy, (1-6C)alkyle ;
R7 est un halogène, méthyle, cyclopropyle, CN, OH, méthoxy, trifluorométhyle, éthylényle, acétylényle, trifluorométhoxy, NO2, NH2, NMe2
R1, R2, R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
R1 et R2 ou R8 et R9 ou R10 et R11 peuvent former un cycle à 5 ou 6 chaînons ayant ou non un hétéroatome tel que O, S, N ;
leurs racémiques, énantiomères, diastéréoisoméres et leurs mélanges, leurs tautomères et leurs sels pharmaceutiquement acceptables.

De manière préférée; la présente invention concerne des dérivés de formule (I) dans laquelle :
R3 est un radical (1-6C)alkyle, aryle, aryle(1-6C)alkyle, hétéroaryle(1-6C)alkyle, hétérocycloalkyle, cycloalkyle, adamantyle, polycycloalkyles, alkényle, alkynyle, CONR1R2, CSNR1R2, COOR1, SO2R1, C(=NH)NR1 ; ces radicaux étant éventuellement substitués par 1 ou plusieurs substituants choisis parmi halogène, CN,
NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle;
R6, R7 sont indépendamment l'un de l'autre un halogène, méthyle, cyclopropyle, CN, OH, méthoxy, trifluorométhyle, éthylényle, acétylényle, trifluorométhoxy, NO2, NH2, NMe2
R1, R2 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ;
R1 et R2 peuvent former un cycle à 5 ou 6 chaînons ayant ou non un hétéroatome tel que O, S, N ;
leurs racémiques, énantiomères, diastéréoisomères et leurs mélanges, leurs tautomères et leurs sels pharmaceutiquement acceptables.

De manière préférée, la présente invention concerne des dérivés de formule (I) dans laquelle :
R3 est un radical (1-6C)alkyle, aryle, aryle(1-6C)alkyle, hétéroaryle(1-6C)alkyle, hétérocycloalkyle, cycloalkyle, adamantyle, polycycloalkyles, alkényle, alkynyle, CONR1R2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle;
R6 est un halogène, méthyle, cyclopropyle, CN, OH, méthoxy, trifluorométhyle, éthylényle, acétylényle, trifluorométhoxy, NO2, NH2, NMe2 ;
R7 est un halogène
R1, R2 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle; CONH₂, formyle, oxo, trifluorométhyle, trifluorométhoxy ;
R1 et R2 peuvent former un cycle à 5 ou 6 chaînons ayant ou non un hétéroatome tel que O, S, N ;
leurs racémiques, énantiomères, diastéréoisomères et leurs mélanges, leurs tautomères et leurs sels pharmaceutiquement acceptables.

Dans les définitions précédentes et celles qui suivent, les radicaux alkyles (1-6C) contiennent 1 à 6 atomes de carbone en chaîne droite ou ramifiée; les radicaux alkényles contiennent 2 à 6 atomes de carbone et une à 3 doubles liaisons conjuguées ou non en chaîne droite ou ramifiée ; les radicaux alkynyles contiennent 2 à 6 atomes de carbone et 1 à 3 triples liaisons conjuguées ou non en chaîne droite ou ramifiée ; les radicaux aryles sont choisis parmi phényle, naphtyle ou indényle ; les radicaux hétéroaryles contiennent 3 à 10 chaînons, contenant éventuellement un ou plusieurs hétéroatomes choisis parmi oxygène, soufre et azote en particulier, thiazolyle, thiényle, pyrrolyle, pyridinyle, furyle, imidazolyle, oxazolyle, pyrazinyle, tetrazolyle, oxadiazolyl, thiadiazolyle, isoxadiazolyl, isothiadiazolyl, isothiazolyle, isoxazolyle, triazolyle, pyrazolyle indolyle; le radical halogène est soit, chlore, iode, fluor, brome ; les radicaux polycycloalkyles sont choisis parmi adamantyle, quinuclidinyle, bornanyle, norbomanyle, bornenyle, norbomenyle ; les radicaux hétéroaryles fusionnés à un cycloalkyle (1-10C) sont choisi parmi indanyle, isochromanyle, chromanyle, 1,2,3,4-tétrahydroisoquinolyle, 1,2,3,4-tétrahydroquinolyle ; les radicaux hétérocycles contiennent 1 à 2 hétéroatomes choisis parmi oxygène, soufre, azote et représentent en particulier piperidinyle, morpholinyle, pyrrolidinyle, imidazolidinyle, pyrrazolidinyle, isothiazolidinyle, thiazolidinyle, isoxazolidinyle, oxazolidinyle, piperazinyle, azétidinyle, 2-piperidone, 3-piperidone, 4-piperidone, 2-pyrrolidone, 3-pyrrolidone.

Les composés de formule (I) présentent un ou plusieurs carbones asymétriques et peuvent donc se présenter sous forme d'isomères, de racémique, d'énantioméres et de diastéréoisomères; ceux-ci font également partie de l'invention ainsi que leurs mélanges.

Parmi les composés de formule (I) utiles selon l'invention on peut citer les composés suivants:
N-(bicyclo[2,2,1]hept-5-en-2ylmethyl)-6-chloro-7-fluoro-5-phenyl-1H-indazol-3-amine
6-chloro-7-fluoro-N-(3,3-dimethylbutyl)-5-phenyl-1H-indazol-3-amine
6-chloro-7-fluoro-N-(3-phenylpropyl)-5-phenyl-1H-indazol-3-amine
6-chloro-7-fluoro-N-(cyclopropylmethyl)-5-phenyl-1H-indazol-3-amine
6-chloro-7-fluoro-N-(cyclopentylmethyl)-5-phenyl-1H-indazol-3-amine
6-chloro-7-fluoro-N-[3-(methylthio)propyl]-5-phenyl-1H-indazol-3-amine
6-chloro-7-fluoro-N-(phenylethyl)-5-phenyl-1H-indazol-3-amine
6-chloro-7-fluoro-N-(cyclohexylmethyl)-5-phenyl-1H-indazol-3-amine
6-chloro-7-fluoro-N-propyl-5-phenyl-1H-indazol-3-amine
6-chloro-7-fluoro-N-(2,2,3,3,4,4,4-heptafluorobutyl)-5-phenyl-1H-indazol-3-amine, hydrate
6-chloro-7-fluoro-N-(4,4,4-trifluorobutyl)-5-phenyl-1H-indazol-3-amine
6-chloro-7-fluoro-N-[(4-methoxyphenyl)methyl]-5-phenyl-1H-indazol-3-amine
6-chloro-7-fluoro-N-(phenylmethyl)-5-phenyl-1H-indazol-3-amine
6-chloro-7-fluoro-N-[(4-cyanophenyl)methyl]-5-phenyl-1H-indazol-3-amine
N-[(4-chlorophenyl)methyl]-6-chloro-7-fluoro-5-phenyl-1H-indazol-3-amine
6-chloro-7-fluoro-N-[(3-methoxyphenyl)methyl]-5-phenyl-1H-indazol-3-amine
6-chloro-7-fluoro-N-[[4-(trifluoromethoxy)phenyl]methyl]-5-phenyl-1H-indazol-3-amine
N-[4-[[[6-chloro-7-fluoro-5-phenyl-1H-indazol-3-yl]amino]methyl]phenyl]-acetamide
6-chloro-7-fluoro-N-[(3,5-dichlorophenyl)methyl]-5-phenyl-1H-indazol-3-amine
6-chloro-7-fluoro-5-phenyl-N-[[4-(trifluoromethyl)phenyl]methyl]-1H-indazol-3-amine
6-chloro-7-fluoro-N-[(4-fluorophenyl)methyl]-5-phenyl-1H-indazol-3-amine
6-chloro-7-fluoro-N-[3-(4-methylphenoxy)phenylmethyl]-5-phenyl-1H-indazol-3-amine
N-(2,2,3,3,4,4,4-heptafluorobutyl]-6-chloro-7-fluoro-5-phenyl-1H-indazol-3-amine
6-chloro-7-fluoro-5-phenyl-N-[[3,5-bis(trifluoromethyl)phenyl]methyl]-1H-indazol-3-amine
6-chloro-7-fluoro-5-phenyl-N-[[3-(trifluoromethyl)phenyl]methyl]-1H-indazol-3-amine
6-chloro-7-fluoro-N-[(6-methoxy-2-naphthalenyl)methyl]-5-phenyl-1H-indazol-3-amine
6-chloro-7-fluoro-N-[(pentafluorophenyl)methyl]-5-phenyl-1H-indazol-3-amine
6-chloro-7-fluoro-N-[[4-(methylthio)phenyl]methyl]-5-phenyl-1H-indazol-3-amine
N-[(4-chloro-3-fluorophenyl)methyl]-6-chloro-7-fluoro-5-phenyl-1H-indazol-3-amine
6-chloro-7-fluoro-5-phenyl-N-(3,3,3-trifluoropropyl)-1H-indazol-3-amine
6-chloro-7-fluoro-5-phenyl-N-(3-thienylmethyl)-1H-indazol-3-amine
N-(bicyclo[2,2,1]hept-5-en-2ylmethyl)-6-chloro-7-fluoro-5-phenyl-1H-indazol-3-amine
N-([1,1'-biphenyl]-4-ylmethyl)-6-chloro-7-fluoro-5-phenyl-1H-indazol-3-amine
6-chloro-7-fluoro-N-[[4-(dimethylamino)phenyl]methyl]-5-phenyl-1H-indazol-3-amine
N-([2,2'-bithiophen]-5-ylmethyl)-6-chloro-7-fluoro-5-phenyl-1H-indazol-3-amine
6-chloro-7-fluoro-5-phenyl-N-[[1-(phenylmethyl)-1H-imidazol-2-yl]methyl]-1H-indazol-3-amine
6-chloro-7-fluoro-N-[[1-methyl-1H-imidazol-2-yl]methyl]-5-phenyl-1H-indazol-3-amine
6-chloro-1-fluoro-N-[(1-methyl-1H-indol-3-yl)methyl]-5-phenyl-1H-indazol-3-amine
6-chloro-7-fluoro-N-[(5-methyl-2-furanyl)methyl]-5-phenyl-1H-indazol-3-amine
6-chloro-7-fluoro-5-phenyl-N-(1H-pyrrol-2-ylmethyl)-1H-indazol-3-amine
6-chloro-7-fluoro-5-phenyl-N-(1H-imidazol-2-yl)methyl]-1H-indazol-3-amine
6-chloro-7-fluoro-5-phenyl-N-(1H-imidazol-4-yl)methyl]-1H-indazol-3-amine
6-chloro-7-fluoro-5-phenyl-N-(1H-pyrazol-3-ylmethyl)-1H-indazol-3-amine
6-chloro-7-fluoro-N-[[2-methyl-1H-imidazol-4-yl]methyl]-5-phenyl-1H-indazol-3-amine
6-chloro-7-fluoro-N-[(3,5-dimethyl-1-phenyl-1H-pyrazol-4-yl)methyl]-5-phenyl-1H-indazol-3-amine
6-chloro-7-fluoro-5-phenyl-N-[[2-phenyl-1H-imidazol-4-yl]methyl]- 1H-indazol-3-amine
6-chloro-7-fluoro-N-[[5-(4-chlorophenyl)-2-furanyl]methyl]-5-phenyl-1H-indazol-3-amine
6-chloro-7-fluoro-5-phenyl-N-[(1-methyl-1H-pyrrol-2-yl)methyl]-1H-indazol-3-amine
4-[5-[[[6-chloro-7-fluoro-5-phenyl-1H-indazol-3-yl]amino]methyl]-2-furanyl]-Benzenesulfonamide
6-chloro-7-fluoro-5-phenyl-N-(3-thienylmethyl)-1H-indazol-3-amine
6-chloro-7-fluoro-5-phenyl-N-[[2-phenyl-1H-imidazol-4-yl]methyl]-1H-indazol-3-amine
2-[[[6-chloro-7-fluoro-5-phenyl-1H-indazol-3-yl]amino]methyl]-5-(methylthio)-1H-imidazole-4-carboxylate d'éthyle
6-chloro-7-fluoro-5-phenyl-N-[[5-[4-(trifluoromethyl)phenyl]-2-furanyl]methyl]-1H-indazol-3-amine
6-chloro-7-fluoro-5-phenyl-N-[2-(1-piperidinyl)ethyl]-1H-indazol-3-amine
6-chloro-7-fluoro-N-[2-(4-morpholinyl)ethyl]-5-phenyl-1H-indazol-3-amine
N-(6-chloro-7-fluoro-5-phenyl-1H-indazol-3-yl)-N'-(3,5-dichlorophenyl)- Urée
N-(6-chloro-7-fluoro-5-phenyl-1H-indazol-3-yl)-N'-(2-propenyl)- Urée
N-(6-chloro-7-fluoro-5-phenyl-1H-indazol-3-yl)-N'-(phenylmethyl)- Urée
N-(6-chloro-7-fluoro-5-phenyl-1H-indazol-3-yl)-N'-4-(phenoxyphenyl)- Urée
N-(6-chloro-7-fluoro-5-phenyl-1H-indazol-3-yl)-N'-[(4-methoxyphenyl)methyl]-Urée
N-(6-chloro-7-fluoro-5-phenyl-1H-indazol-3-yl)-N'-[4-(trifluoromethyl)phenyl]- Urée
N-(6-chloro-7-fluoro-5-phenyl-1H-indazol-3-yl)-N'-(4-methoxyphenyl)- Urée
N-(6-chloro-7-fluoro-5-phenyl-1H-indazol-3-yl)-N-cyclohexyl- Urée
N-(6-chloro-7-fluoro-5-phenyl-1H-indazol-3-yl)-N'-propyl- Urée
N-(6-chloro-7-fluoro-5-phenyl-1H-indazol-3-yl)-N-(4-chlorophenyl)- Urée
N-(6-chloro-7-fluoro-5-phenyl-1H-indnol-3-yl)-N'-(4-fluorophenyl)- Urée
N-[6-chloro-7-fluoro-5-phenyl-1H-indazol-3-yl]-N-tricyclo[3.3.1.13,7]dec-1-yl-Urée
N-(6-chloro-7-fluoro-5-phenyl-1H-indazol-3-yl)-N-(4-methylphenyl)- Urée
N-(6-chloro-7-fluoro-5-phenyl-1H-indazol-3-yl)-Urée
N-(6-ehloro-7-methyl-5-phenyl-1H-indazol-3-yl)-Urée
N-(6-chloro-7-cyano-5-phenyl-1H-indazol-3-yl)-Urée
N-(6-chloro-7-cyclopropyl-5-phenyl-1H-indazol-3-yl)-Urée
N-(6-chloro-7-hydroxy-5-phenyl-1H-indazol-3-yl)-Urée
N-(6-chloro-7-methoxy-5-phenyl-1H-indazol-3-yl)-Urée
N-(6-chloro-7-trifluoromethyl-5-phenyl-1H-indazol-3-yl)-Urée
N-(6-chloro-7-trifluoromethoxy-5-phenyl-1H-indazol-3-yl)-Urée
N-(6-chloro-7-nitro-5-phenyl-1H-indazol-3-yl)-Urée
N-(6-chloro-7-amino-5-phenyl-1H-indazol-3-yl)-Urée
N-(6-chloro-7-dimethylamino-5-phenyl-1H-indazol-3-yl)-Urée
N-(6-chloro-7-ethynyl-5-phenyl-1H-indazol-3-yl)-Urée
N-[6-chloro-7-fluoro-5-phenyl-1H-indazol-3-yl]-4-methyl-Benzenesulfonamide
N-[6-chloro-7-fluoro-5-phenyl-1H-indazol-3-yl]-Methanesulfonamide
N-[6-chloro-7-fluoro-5-phenyl-1H-indazol-3-yl]-2-propanesulfonamide
N-[6-chloro-7-fluoro-5-phenyl-1H-indazol-3-yl]-2,2,2-trifluoro-ethanesulfonamide
N-[6-chloro-7-fluoro-5-phenyl-1H-indazol-3-yl]-2-thiophenesulfonamide
N-[6-chloro-7-fluoro-5-phenyl-1H-indazol-3-yl]- Benzenesulfonamide
N-[6-chloro-7-fluoro-5-phenyl-1H-indazol-3-yl]-4-(trifluoromethyl)-Benzenesulfonamide
N-[6-chloro-7-fluoro-5-phenyl-1 H-indazol-3-yl]-5-(3-isoxazolyl)-2-thiophenesulfonamide
N-[6-chloro-7-fluoro-5-phenyl-1 H-indazol-3-yl]-4-fluoro-Benzenesulfonamide
N-[6-chloro-7-fluoro-5-phenyl-1H-indazol-3-yl]-4-methoxy-Benzenesulfonamide
N-[6-chloro-7-fluoro-5-phenyl-1H-indazol-3-yl]-Benzenemethanesulfonamide
N-[6-chloro-7-fluoro-5-phenyl-1H-indazol-3-yl]-1-methyl-1H-imidazole-4-sulfonamide
N-[6-chloro-7-fluoro-5-phenyl-1H-indazol-3-yl]-4-(1,1-dimethylethyl)-Benzenesulfonamide
N-[4-[[(6-chloro-7-fluoro-5-phenyl-1H-indazol-3-yl)amino]sulfonyl]phenyl]-Acetamide
N-[6-chloro-7-fluoro-5-phenyl-1H-indazol-3-yl]-4-methyl-Benzenemethanesulfonamide
6-chloro-7-fluoro-N-(pentafluorophenyl)-5-phenyl-1H-indazol-3-amine
6-chloro-7-fluoro-N-(3,4-difluorophenyl)-5-phenyl-1H-indazol-3-amine
6-chloro-7-fluoro-5-phenyl-N-(2,3,5,6-tetrafluorophenyl)-1H-indazol-3-amine
6-chloro-7-fluoro-5-phenyl-N-(2,4,6-trifluorophenyl)-1H-indazol-3-amine
6-chloro-7-fluoro-N-(4-fluorophenyl)-5-phenyl-1H-indazol-3-amine
6-chloro-7-fluoro-N-[3-(trifluoromethyl)phenyl]-5-phenyl-1H-indazol-3-amine
6-chloro-7-fluoro-N-[4-(trifluoromethyl)phenyl]-5-phenyl-1H-indazol-3-amine
6-chloro-7-fluoro-N-[3-fluoro-5-(trifluoromethyl)phenyl]-5-phenyl-1H-indazol-3-amine
6-chloro-7-fluoro-N-(4-nitrophenyl)-5-phenyl-1H-indazol-3-amine
6-chloro-7-fluoro-N-(3-nitrophenyl)-5-phenyl-1H-indazol-3-amine
6-chloro-7-fluoro-N-(3-methoxyphenyl)-5-phenyl-1 H-indazol-3-amine
6-chloro-7-fluoro-N-(4-methoxyphenyl)-5-phenyl-1 H-indazol-3-amine
6-chloro-7-fluoro-N,5-diphenyl-1H-indazol-3-amine
6-chloro-7-fluoro-N-(1-pyridinyl)-5-phenyl-1H-indazol-3-amine
6-chloro-7-fluoro-N-(2-pyridinyl)-5-phenyl-1H-indazol-3-amine
N-butyl-6-chloro-7-fluoro-5-phenyl-1H-indazol-3-amine
N-(6-chloro-7-fluoro-5-phenyl-1H-indazol-3-yl)-N'-phenyl- Urée
N-(6-chloro-7-fluoro-5-phényl-1H-indazol-3-yl)-3-méthoxy-benzènesulfonamide
leurs isomères, leurs mélanges, leurs racémiques énantiomères, diastéréoisomères, tautomères ainsi que leurs sels pharmaceutiquement acceptables, et plus particulièrement le composé suivant :
Piperidine-1-carboxylic acid (6,7-difluoro-5-phenyl-1H-indazol-3-yl)-amide
Pyrrolidine-1-carboxylic acid (6,7-difluoro-5-phenyl-1H-indazol-3-yl)-amide
1-(6,7-Difluoro-5-phenyl-1H-indazol-3-yl)-3-[3-(4-methyl-piperazin-1-yl)-propyl]-urea
N-(6,7-difluoro-5-phenyl-1H-indazol-3-yl)-N'-phenyl- Urée
ses tautomères ainsi que leurs sels pharmaceutiquement acceptables.

L'invention concerne également les compositions pharmaceutiques contenant en tant que principe actif un dérivé de formule (I) dans laquelle
R3 est un radical (1-6C)alkyle, aryle, aryle(1-6C)alkyle, hétéroaryle(1-6C)alkyle, hétérocycloalkyle, cycloalkyle, adamantyle, polycycloalkyles, alkényle, alkynyle, CONR1R2, CSNR1R2, CSNR1R2, COOR1, SO2R1, C(=NH)R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, hétérocycle, formyle, trifluorométhyle, trifluorométhylsulfanyle, trifluorométhoxy, (1-6C)alkyle ;
R5, R6, sont indépendamment l'un de l'autre choisis parmi les radicaux suivant halogène, CN, NO2, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle, adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy, (1-6C)alkyle ;
R7 est un halogène, méthyle, cyclopropyle, CN, OH, méthoxy, trifluorométhyle, éthylényle, acétylényle, trifluorométhoxy, NO2, NH2, NMe2
R1, R2, R8, R9, R10, R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
R1 et R2 ou R8 et R9 ou R10 et R11 peuvent former un cycle à 5 ou 6 chaînons ayant ou non un hétéroatome tel que O, S, N;
leurs racémiques, énantiomères, diastéréoisomères et leurs mélanges, leurs tautomères et leurs sels pharmaceutiquement acceptables.

De manière préférée, la présente invention concerne les compositions pharmaceutiques contenant en tant que principe actif un dérivé de formule (I) dans laquelle:
R3 est un radical (1-6C)alkyle, aryle, aryle(1-6C)alkyle, hétéroaryle(1-6C)alkyle, hétérocycloalkyle, cycloalkyle, adamantyle, polycycloalkyles, alkényle, alkynyle, CONRIR2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle;
R6, R7 sont indépendamment l'un de l'autre un halogène, méthyle, cyclopropyle, CN, OH, méthoxy, trifluorométhyle, éthylényle, acétylényle, trifluorométhoxy, NO2, NH2, NMe2
R1, R2 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ;
R1 et R2 peuvent former un cycle à 5 ou 6 chaînons ayant ou non un hétéroatome tel que O, S, N ;
leurs racémiques, énantiomères, diastéréoisomères et leurs mélanges, leurs tautomères et leurs sels pharmaceutiquement acceptables.

La présente invention concerne également l'utilisation à titre de médicament des dérivés d'aminoindazoles de formule (I) dans laquelle :
R3 est un radical (1-6C)alkyle, aryle, aryle(1-6C)alkyle, hétéroaryle(I-6C)alkyle, hétérocycloalkyle, cycloalkyle, adamantyle, polycycloalkyles, alkényle, alkynyle, CONR1R2, CSNR1R2, CSNR1R2, COOR1, SO2R1, C(=NH)R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, hétérocycle, formyle, trifluorométhyle; trifluorométhylsulfanyle, trifluorométhoxy, (1-6C)alkyle :
R5, R6, sont indépendamment l'un de l'autre choisis parmi les radicaux suivant halogène, CN, NO2, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle, adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formule, trifluorométhyle, trifluorométhoxy, (1-6C)alkyle ;
R7 est un halogène, méthyle, cyclopropyle, CN, OH, méthoxy, trifluorométhyle, éthylényle, acétylényle, trifluorométhoxy, NO2, NH2, NMe2
R1, R2, R8, R9, R10, R11 sont indépendamment l'un de l'autre un hydrogène, (1-6C)alkyle; aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formule, trifluorométhyle, trifluorométhoxy ;
R1 et R2 ou R8 et R9 ou R10 et R11 peuvent former un cycle à 5 ou 6 chaînons ayant ou non un hétéroatome tel que O, S, N ;
leurs racémiques, énantiomères, diastéréoisomères et leurs mélanges, leurs tautomères et leurs sels pharmaceutiquement acceptables.

De manière préférée, la présente invention concerne l'utilisation à titre de médicament des dérivés d'aminoindazoles de formule (I) dans laquelle :
R3 est un radical (1-6C)alkyle, aryle, aryle(1-6C)alkyle, hétéroaryle(1-6C)alkyle, hétérocycloalkyle, cycloalkyle, adamantyle, polycycloalkyles, alkényle, alkynyle, CONR1R2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle;
R6, R7 sont indépendamment l'un de l'autre un halogène, méthyle, cyclopropyle, CN, OH, méthoxy, trifluorométhyle, éthylényle, acétylényle, trifluorométhoxy, NO2, NH2, NMe2
R1, R2 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ;
R1 et R2 peuvent former un cycle à 5 ou 6 chaînons ayant ou non un hétéroatome tel que O, S, N ;
leurs racémiques, énantiomères, diastéréoisomères et leurs mélanges, leurs tautomères et leurs sels pharmaceutiquement acceptables.

Les dérives de formule (I) peuvent être obtenus à partir des dérivés 3-amino correspondants (V), pour lesquels l'azote en 1 est eventuellement protégé avec un groupement Pr. Pr est un radical triméthylsilyléthoxyméthyle, tosyle, mésyle, benzyle ou les groupements connus pour la protection des NH- d'hétérocycles aromatiques comme indiqués dans T.W. GREENE, Protective groups in organic Synthesis, J. Wiley-Interscience Publication (1999)

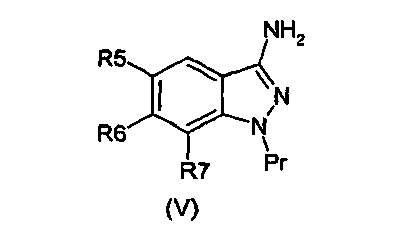

Les 3-amino 1H-indazoles de formule (II) peuvent être obtenus par réaction d'un 2-fluorobenzonitrile avec de l'hydrazine, hydrate ou chlorhydrate au reflux de 2 à 18 heures dans un alcool type éthanol ou n-butanol selon (R.F. KALTENBACH, Bioorg. Med. Chem. Lett., 9, (15), 2259-62, (1999)):

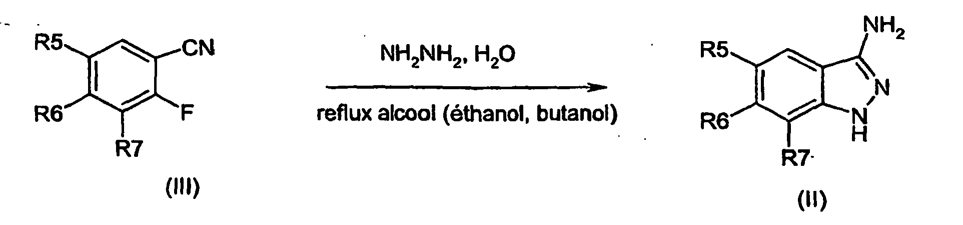

Pour les composés pour lesquels R5, R6 sont indépendamment l'un de l'autre choisis parmi les radicaux suivant halogène, CN, NO₂, NH₂, OH, COOH, C(O)OR8, -OC(O)R8, NR8R9, NHC(O)R8, C(O)NR8R9, NHC(S)R8, C(S)NR8R9, SR8, S(O)R8, SO₂R8, NHSO₂R8, SO₂NRBR9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, cycloalkyle, alkényle, alkynyl, adamantyle; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, oxo, trifluorométhyle, trifluorométhoxy, (1-6C)alkyle; peuvent être obtenus par des réactions mettant en jeu la chimie du palladium : Suzuki, (A. SUZUKI, Pure Appl. Chem. 63, 419-22, (1991), Stille (J., STILLE, Angew. Chem. Int. Ed. 25, 508-24, (1986), Heck, (R. F. HECK, Org. React., 27, 345-90, (1982), Sonogashira, ( K. SONOGASHIRA, Synthesis 777, (1977), Buchwald (S.L. BUCHWALD, Acc. Chem.Re., 31, 805, (1998) à partir des dérivés halogénés correspondants.

Pour cela il est nécessaire de protéger les fonctions réactives. Ainsi, les fonctions OH, SH, COOH, NH2 doivent être protégées avant de faire le couplage. Les groupements protecteurs sont introduits selon toutes les méthodes connues de l'homme de l'art et notamment celles décrites par T.W. GREENE, Protective groups in Organic Synthesis, J. Wiley-Interscience Publication (1999). Il est préférable de protéger l'azote en position 1 par des groupements tels que le *tert*-butoxycarbonyle ou des dérivés siliciés. On choisira de préférence un groupement silylé *tert-*butyldiméthylsilyle, triisopropylsilyle qui peuvent être éliminés par les anions fluorures ou avec l'acide acétique et plus particulièrement un groupement triméthylsilyléthoxyméthyle clivable par le fluorure de tétrabutylammonium au reflux dans des solvants tels que le tétrahydrofurane, le dioxane (J. P. WHITTEN, J. Org. Chem., 51, 1891, (1986) ; B. H. LIPSHUTZ, Tetrahedron Lett., 4095, (1986)) ou par l'acide chlorhydrique 2N dans le methanol ou l'ethanol au reflux.

Les dérivés protégés en 1 par triméthylsilyléthoxyméthyle sont obtenus en faisant réagir le composés de départ avec le chlorure de triméthylsilyléthoxyméthyle en présence d'hydrure de sodium dans un solvant tel que le diméthylformamide à température ambiante (J. P. WHITTEN, J. Org. Chem., 51, 1891, (1986) ; M. P. EDWARDS, Tetrahedron, 42, 3723, (1986))

De même, la fonction azote 1-NH de l'indazole sera protégée par des groupements tels tosyle, carbamate, benzyle ou dérivés silylés. Par exemple dans le cas où l'on voudrait pratiquer un couplage au palladium sur un dérivé halogéné en position 6, il faudra protéger l'azote en position 1 comme montré ci-dessous (X = Cl, Br, I) :

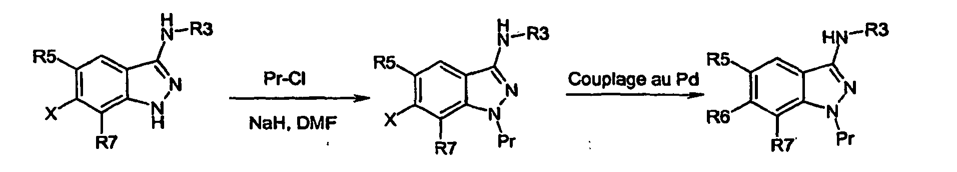

La déprotection s'effectue selon des méthodes connues par l'homme du métier et décrites par T.W. GREENE, Protective groups in Organic Synthesis, J. Wiley-Interscience Publication (1999). Par exemple, si le groupement protecteur en position 1 est un triméthylsilyléthoxyméthyle il pourra être déprotégé par réaction avec le fluorure de tétrabutylammonium comme montré ci-dessous:

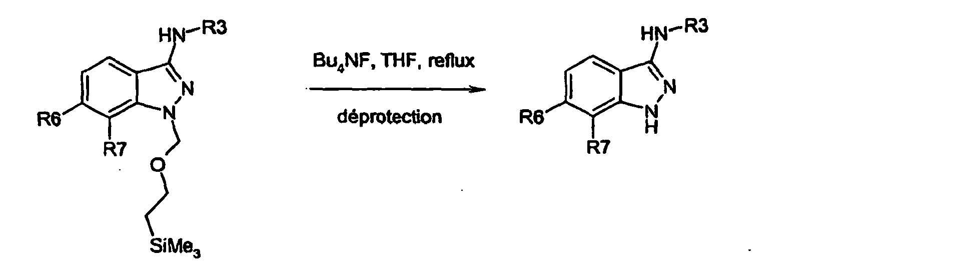

Lorsque l'un des groupements R5, R6 engagé pour le couplage utilisant la chimie du palladium contient lui-même une fonction réactive telle hydroxyle, amine, thiol, acide ou de manière générale renferme un hétéroatome, il est nécessaire de protéger ces dernières également avant d'effectuer le couplage au palladium. Ainsi par exemple une fonction phénol sera introduite sous la forme protégée (O-benzyle par exemple) à partir du dérivé chloré et l'azote en 1 étant protégé comme explicité auparavant :

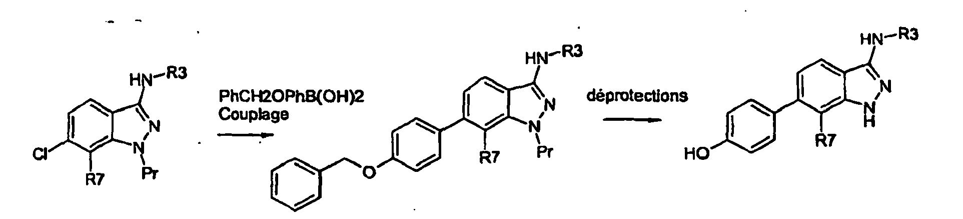

Le groupement benzyle sera ensuite éliminé par exemple par traitement à l'iodure de triméthylsilyle au reflux dans l'acétonitrile. La protection pourra également être réalisée par un groupement triméthylsilyléthoxyméthyle clivable par le fluorure de tétrabutylammonium au reflux dans des solvants tels que le tetrahydrofurane, le dioxane. (J. P. WHITTEN, J. Org. Chem., 51, 1891, (1986) ; B. H. LIPSHUTZ, Tetrahedron Lett., 4095, (1986)). ou par l'acide chlorhydrique 2N dans le methanol ou l'ethanol au reflux.

Lorsque R5 et R6 sont indépendamment l'un de l'autre un aryle et un halogène, la fonction aryle est introduite à partir d'un couplage au palladium sur une position bromée, l'azote en 1 et 3 étant protégé de manière appropriée. De préférence Pr représente un triméthylsilyléthoxyméthyle et Pr' représente un groupement n-butylcarboxy qui forme avec l'azote un n-butylamide. L'étape de déprotection de l'amide se fait en présence d'éthanolamine à reflux pendant une semaine dans la DMF. Ce clivage peut être aussi réalisé par le chlorure stanneux dans l'ethanol ( R J Griffin, J.Chem.Soc. Perkin I 1992, 1811-1819) ou bien le methylate de sodium dans le methanol (Y. Furukawa, Chem.Pharm.Bull. 1968,16, 1076) ou tout autre alcoolate dans l'alcool correspondant.

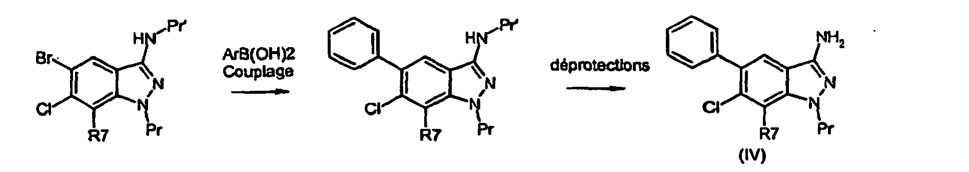

Lorsque R5 et R6 sont indépendamment l'un de l'autre un aryle et un halogène, la fonction aryle est introduite à partir d'un couplage au palladium sur une position bromée, l'azote en 1 et 3 étant protégé de manière appropriée. De préférence Pr représente un triméthylsilyléthoxyméthyle et Pr' représente un groupement n-butylcarboxy qui forme avec l'azote un n-butylamide. La subsitution électrophile est réalisée avec du nitronium tetrafluoroborate (NO2BF4) par exemple. Le couplage de la position 5 se fait à partir de la chimie du palladium (couplage de Suzuki, Heck, Sonogashira). La position 7 est fonctionnalisée en fonction des substituants recherchés par des réductions, halogénations pour introduire un brome, couplage avec la chimie du palladium (couplage de Suzuki, Heck, Sonogashira) pour introduire des fonctions aryle, hétéroaryle, alkyle, alkényle, alkynyle, acétylenique. L'étape de déprotection de l'amide se fait en présence d'éthanolamine à reflux pendant une semaine dans la DMF. Ce clivage peut être aussi réalisé par le chlorure stanneux dans l'ethanol ( R J Griffin, J.Chem.Soc. Perkin I 1992, 1811-1819) ou bien le methylate de sodium dans le ethanol (Y. Furukawa, Chem.Pharm.Bull. 1968,16, 1076) ou tout autre alcoolate dans l'alcool correspondant. La déprotection en 3 permet d'obtenir la fonction NH2 qui peut réagir avec les groupements nécessaires pour introduire les substitutions recherchées en 3 telle que décrite dans les pages suivantes.

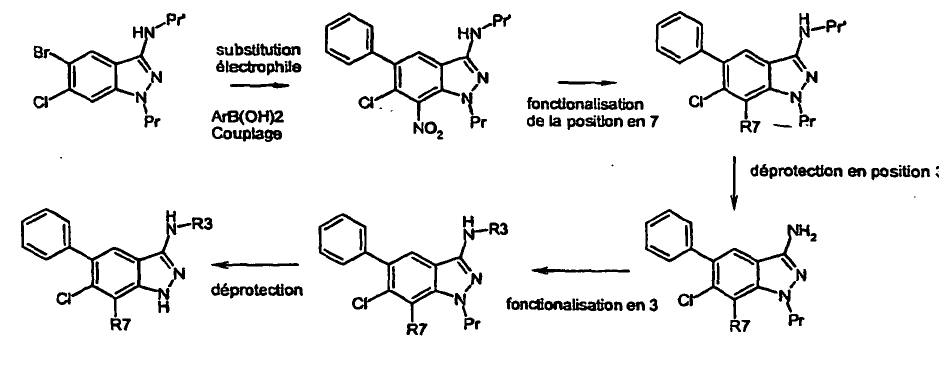

Les composés de formule II sont le point de départ pour l'obtention d'une grande variété de produits obtenus par réaction de la fonction amine primaire du 3-amino indazole dans toutes les réactions classiques de cette fonction telles que : alkylation, acylation, reactions avec les dérivés carbonylés suivit de réduction, sulfonation, transformation en urées ou carbamates, arylation ( réactions de Castro ou de Buchwald) etc...

Les amino reduction de dérivés formule générale (I) où R3 est H lorsque Pr est triméthylsilyléthoxyméthyle peuvent être réalisées à l'aide de dérivés du bore comme le triacétoxyborohydrure de sodium dans du dichlorométhane en présnece d'un aldéhyde de type R1CHO dans les conditions décrites dans Organic Reactions vol 59 1-714 ( E.Baxter, A.Reitz) ou par les autres reducteurs couramment utilisés pour réduire les imines pour former des produits où R3 est (1-6C)alkyle, aryle(1-6C)alkyle, hétéroaryle(1-6C)alkyle, hétérocycloalkyle, cycloalkyle, polycycloalkyles, ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle.

Les condensations avec des dérivés formule générale (I) où R3 est H sur les isocyanates de type OCNR1 peuvent notamment être réalisées dans du tétrahydrofurane et selon les exemples décrits dans Comprehensive Organic functionnal Group Transformations vol 6 (Katritzky, Meth-Cohn, Rees 1995) pour former des produits où R3 est CONR1R2, CSNR1R2, R1, R2, sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy.

Les sulfonations de dérivés de formule générale (I) où R3 est H peuvent être réalisées à partir d'un chorure de sulfonyle de type R1SO2C1, en présence d'une base (en particulier les amines tertiaires comme la triéthylamine ou aromatiques comme la pyridine) dans un solvant usuel comme par exemple le dichlorométhane pour former les produits où R3 est SO2R1 et R1 est un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy.

Les composés de formule (I) sont isolés et peuvent être purifiés par les méthodes connues habituelles, par exemple par cristallisation, chromatographie ou extraction. Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, maléate, iséthionate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de kinase et sont ainsi utiles pour la prévention et le traitement les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, l'hypertension essentielle, les maladies cardiovasculaires athérosclérotiques, le syndrome des ovaires polycystiques, le syndrome X, l'immunodéficience et le caner.

Leurs activités ont été déterminées en mesurant l'inhibition de la phosphorylation de la protéine tau dans les coupes de cortex de rat adulte.

Les coupes de cortex d'une épaisseur de 300µm sont préparées à partir de rats mâles OFA (Iffa-Credo) âgés de 8-10 semaines, sacrifiés par décapitation. Elles sont incubées dans 5 ml de milieu DMEM contenant du pyruvate et du glucose 4.5 g/l à 37°C pendant 40 min. Les coupes sont ensuite lavées 2 fois avec le milieu, distribuées dans des microtubes (50µl dans 500µl de milieu avec ou sans composés à tester), et incubées à 37°C sous agitation. Deux heures plus tard, l'expérience est arrêtée par centrifugation. Les coupes sont lysées, sonifiées et centrifugées à 18300g, 15 min à 4°C. La concentration en protéines du surnageant est déterminée par un dosage commercial (BCA Protein Assay , Pierce) basé sur la méthode de Lowry.

Les échantillons, dénaturés au préalable 10 min à 70°C, sont séparés sur gel vertical 4-12%Bis-Tris en présence de tampon MOPS-SDS et électrotansferrés sur membrane de nitrocellulose. L'immunomarquage est réalisé par l'anticorps monoclonal AD2 qui reconnaît spécifiquement les épitopes phosphorylés Ser396/404 de la protéine tau. Les protéines immunoréactives sont visualisées par addition d'un deuxième anticorps dirigé contre les IgG de souris et couplé à la peroxydase et d'un substrat chimioluminescent. Les autoradiogrammes obtenus sont enfin quantifiés à l'aide du logiciel 'GeneTools' de Syngene (GeneGnome, Ozyme) pour déterminer une CI50.

Les composés de formule (I) présentent une activité très intéressante et en particulier certains composés ont une CI50 inférieure à 100 µM.

Les exemples suivants illustrent l'invention de manière non limitative.

Les comditions d'analyse des produits en LC/MS ont été réalisées sur un appareil Waters Alliance 2695 pour la partie LC et Waters-Micromass Platform II pour la partie masse.

### Préparation des produits intermédiaires :

### 6,7-difluoro-1H-indazole-3-amine:

A 0.46 cm³ de 2,3,4-trifluorobenzonitrile dans 10 cm³ d'éthanol absolu, on additionne 0.32 cm³ d'hydrazine monohydratée. On chauffe le milieu vers 75°C pendant 17 heures puis on ajoute 10 cm³ d'acétate d'éthyle, 5 cm³ de tétrahydrofurane et 5 cm³ d'eau distillée. La phase organique est décantée et relavée avec 10 cm³ d'eau distillée puis avec 10 cm³ d'une solution aqueuse saturée en chlorure de sodium. La phase organique est décantée, séchée sur sulfate de magnésium, filtrée et concentrée à sec sous pression réduite (2 kPa ; 50°C). Le résidu obtenu est purifié par chromatographie sous pression d'argon de 50 kPa, sur une colonne de gel de silice (granulométrie 40-60 µm ; diamètre 1.5 cm), en éluant avec un mélange cyclohexane-acétate d'éthyle (50/50 en volumes). Les fractions contenant le produit attendu sont réunies puis évaporées sous pression réduite (2 kPa ; 40°C) ; on obtient après séchage (90 Pa ; 40°C), 100 mg de 6,7-difluoro-1H-indazole-3-amine sous forme d'un solide blanc fondant à 183°C .

Spectre de R.M.N. ¹H (300 MHz, (CD₃)₂SO d6, δ en ppm) : 5,57 (mf : 2H) ; 6,93 (mt : 1H) ; 7,52 (ddd, J = 8,5 - 4,5 et 1 Hz : 1H) ; 12,01 (mf : 1H).

### N-(6,7-difluoro-1H-indazol-3-yl)-butanamide:

A 1 g de 6,7-diflucro-1H-indazole-3-amine décrit précédemment, dans 15 cm³ de pyridine, on ajoute 0.61 cm³ de chlorure de butyryle après avoir refroidit vers 3°C puis on laisse à température ambiante pendant 76 heures. Le milieu réactionnel est concentré sous pression réduite (2 kPa ; 40°C) et le résidu est repris par 25 cm³ d'acétate d'éthyle et par 25 cm³ d'eau. La phase organique est lavée avec 25 cm³ d'eau distillée puis avec 25 cm³ d'une solution aqueuse saturée en chlorure de sodium. Après séchage sur sulfate de magnésium, filtration et concentration sous pression réduite (2 kPa ; 40°C), le résidu obtenu est purifié par chromatographie sous pression d'argon de 50 kPa, sur une colonne de gel de silice (granulométrie 40-60 µm ; diamètre 3 cm), en éluant par un mélange dichlorométhane-méthanol (98/2 en volumes). Les fractions contenant le produit attendu sont réunies puis évaporées sous pression réduite (2 kPa ; 40°C) ; on obtient après séchage (90 Pa ; 40°C), 596 mg de N-(6,7-difluoro-1H-indazol-3-yl)-butanamide sous forme d'un solide blanc fondant à 191°C.

Spectre de R.M.N. ¹H (300 MHz, (CD₃)₂SO d6, δ en ppm) : 0,97 (t, J = 7,5 Hz : 3H) ; 1,67 (mt : 2H) ; 2,40 (t, J = 7 Hz : 2H) ; 7,10 (mt : 1H) ; 7,63 (dd large, J = 9 et 4,5 Hz : 1H) ; 10,47 (mf étalé : 1H) ; 13,35 (mf étalé : 1H).

### N-[6,7-difluoro-1-[[2-(triméthylsilyl)éthoxy]méthyl]-1H-indazol-3-yl]-butanamide

A 1.65 g d'hydrure de sodium à 60% dans l'huile, dans 50 cm³ de diméthylformamide on ajoute goutte à goutte une solution de 1.1 g de N-(6,7-difluoro-1H-indazol-3-yl)-butanamide préparé précédemment, dans 180 cm³ de diméthylformamide en 3 heures. Le milieu réactionnel est concentré à sec sous pression réduite et repris avec 250 cm³ d'acétate d'éthyle et 200 cm³ d'eau ; la phase organique est décantée, lavée par 150 cm³ d'eau, séchée sur sulfate de magnésium, filtrée et concentrée à sec sous pression réduite (2 kPa ; 50°C). Le brut est purifié par chromatographie sous pression d'argon de 50 kPa, sur colonne de gel de silice ( granulométrie 40-60 µm ; diamètre 6 cm), en éluant par un mélange cyclohexane-acétate d'éthyle (80/20 en volumes). Les fractions contenant le produit attendu sont réunies et évaporées sous pression réduite (2 kPa ; 50°C) pour donner 7.3 g de N-[6,7-difluoro-1-[[2-(triméthylsilyl)éthoxy]méthyl]-1H-indazol-3-yl]-butanamide sous forme d'huile jaune.

Spectre de R.M.N. ¹H (300 MHz, (CD₃)₂SO d6, δ en ppm) : - 0,09 (s : 9H) ; 0,82 (t, J = 8 Hz : 2H) ; 0,96 (t, J = 7,5 Hz : 3H) ; 1,67 (mt : 2H) ; 2,41 (t, J = 7 Hz : 2H) ; 3,56 (t, J = 8 Hz : 2H) ; 5,66 (s : 2H) ; 7,22 (ddd, J = 11 - 9 et 7 Hz : 1H) ; 7,69 (dd large, J = 9 et 4,5 Hz : 1H) ; 10,60 (mf : 1H).
Spectre de masse : M = 369

### N-[5-bromo-6,7-difluoro-1-[[2-(triméthylsilyl)éthoxy]méthyl]-1H-indazol-3-yl]-butanamide

A 1g de N-[6,7-difluoro-1-[[2-(triméthylsilyl)éthoxy]méthyl]-1H-indazol-3-yl]-butanamide précédemment décrit dans 30 cm³ de chloroforme, on ajoute 0.87 cm³ de pyridine, puis additionne 0.56 cm³ de brome et on chauffe au reflux la nuit. On ajoute au milieu réactionnel 50 cm³ de dichlorométhane et 50 cm³ d'une solution aqueuse de thiosulfate de sodium à 10%. Après 10 minutes d'agitation, l'insoluble est éliminé par filtration sur verre fritté et la phase organique est lavée avec 50 cm³ d'eau et avec 50 cm³ d'une solution saturée en chlorure de sodium. La phase organique est décantée, séchée sur sulfate de magnésium, filtrée et concentrée à sec sous pression réduite (2 kPa ; 45 °C). Le brut, 1.1 g est purifié par chromatographie sous pression d'argon de 50 kPa, sur colonne de gel de silice (granulométrie 40-60 µm ; diamètre 3 cm), en éluant par un mélange cyclohexane-acétate d'éthyle (90/10 en volumes). Les fractions contenant le produit attendu, sont réunies et évaporées sous pression réduite (2 kPa ; 50°C). On obtient après séchage (90 Pa ; 45°C), 230 mg de N-[5-bromo-6,7-difluoro-1-[[2-(triméthylsilyl)éthoxy]méthyl]-1H-indazol-3-yl]-butanamide sous forme d'huile incolore.

Spectre de R.M.N. ¹H (300 MHz, (CD₃)₂SO d6, δ en ppm) : - 0,08 (s : 9H) ; 0,82 (t, J = 8 Hz : 2H) ; 0,96 (t, J = 7,5 Hz : 3H) ; 1,67 (mt : 2H) ; 2,42 (t, J = 7 Hz : 2H) ; 3,55 (t, J = 8 Hz : 2H) ; 5,66 (s : 2H) ; 8,08 (dd, J = 6 et 2 Hz : 1H) ; 10,72 (mf : 1H).
Spectre de masse : M = 447

### N-[6,7-difluoro-5-phényl-1-[[2-(triméthylsilyl)éthoxy]méthyl]-1H-indazol-3-yl]-butanamide

A 1.15 g de N-[5-bromo-6,7-difluoro-1-[[2-(triméthylsilyl)éthoxy]méthyl]-1H-indazol-3-yl]-butanamide préparé précédemment, dans 150 cm³ de dioxane, on ajoute, 469 mg d'acide phénylboronique, 760 mg de carbonate de sodium dans 30 cm³ d'eau et 379 mg de tétrakis-triphénylphosphine palladium et on chauffe au reflux pendant 4 heures. On dilue le milieu réactionnel avec 100 cm³ d'acétate d'éthyle et 75 cm³ d'eau et on filtre sur verre fritté garni de célite. La phase organique est décantée, lavée avec 75 cm³ d'eau et avec 75 cm³ d'une solution saturée en chlorure de sodium, séchée sur sulfate de magnésium, filtrée et concentrée à sec sous pression réduite (2 kPa ; 50°C) pour donner 2 g de brut sous forme d'une huile noire. Le brut est purifié par chromatographie sous pression d'argon de 50 kPa, sur colonne de gel de silice ( granulométrie 40-60 µm ; diamètre 3.5 cm), en éluant avec un mélange cyclohexane-acétate d'éthyle (85/15 en volumes). Les fractions contenant le produit attendu, sont réunies, évaporées sous pression réduite (2 kPa ; 50°C) et séchées (90 Pa, 45°C); pour donner 1.1 g de N-[6,7-difluoro-5-phényl-1-[[2-(triméthylsilyl)éthoxy]méthyl]-1H-indazol-3-yl]-butanamide sous forme d'huile jaune.

Spectre de R.M.N. ¹H (300 MHz, (CD₃)₂SO d6, δ en ppm) : - 0,05 (s : 9H) ; 0,84 (t, J = 8 Hz : 2H) ; 0,95 (t, J = 7,5 Hz : 3H) ; 1,66 (mt : 2H) ; 2,43 (t, J = 7 Hz : 2H) ; 3,59 (t, J = 8 Hz: 2H) ; 5,69 (s : 2H) ; de 7,40 à 7,65 (mt : 5H) ; 7,82 (d large, J = 7 Hz : 1H) ; 10,64 (mf: 1H).
Spectre de masse : M = 445

### N-[6,7-difluoro-5 phenyl-1-[[2-(triméthylsilyl)éthoxy]méthyl]-1H-indazole-3-amine

A 1.6 g de N-[6,7-difluoro-5-phényl-1-[[2-(triméthylsilyl)éthoxy]méthyl]-1H-indazol-3-yl]-butanamide décrit précedemment, dans 50 cm³ de diméthylformamide on ajoute 1.1 cm³ d'éthanolamine puis 1.50 g de carbonate de potassium et on chauffe au reflux pendant une semaine. Le milieu réactionnel est concentré à sec sous pression réduite et repris par 150 cm³ d'acétate d'éthyle et 75 cm³ d'eau. La phase organique est décantée et lavée successivement par 2 fois 75 cm³ d'eau et 50 cm³ de saumure. La phase organique est séchée sur sulfate de magnésium, filtrée puis concentrée à sec sous pression réduite (2 kPa ; 50°C). L'huile brute obtenue est purifiée par chromatographie sous pression d'argon de 50 kPa, sur colonne de gel de silice (granulométrie 40-60 µm ; diamètre 4cm), en éluant par un mélange cyclohexane-acétate d'éthyle (80/20 en volumes). Les fractions contenant le produit attendu, sont réunies et évaporées sous pression réduite (2 kPa ; 50°C). On obtient après séchage (90 Pa ; 45°C), 0,32 g de 6,7-dinuoro-5-phényl-1-[[2-(triméthylsilyl)éthoxy]méthyl]-1H-indazole-3-amine.

### 6,7-difluoro-5-phényl-1H-indazole-3-amine:

A 661 mg de 6,7-difluoro-5-phenyl-1-[[2-(triméthylsilyl)éthoxy]méthyl]-1H-indazole-3-amine dans 15 ml de methanol sont ajoutés 1.1 ml d'HCl 2N. La réaction est placé sous micro ondes 3mn à 140°C. Après hydrolyse avec une solution saturée de KH2PO4 et extraction avec du chlorure de méthylène, on évapore et chromatographie sur silice (chlorure de méthylène/acétate d'éthyle) pour obtenir 314 mg de 6,7-difluoro-5-phényl-1H-indazole-3-amine

### Exemple 1 : Piperidine-1-carboxylic acid (6,7-difluoro-5-phenyl-1H-indazol-3-yl)-amide

### Étape1

A 387.8 mg de compose (6,7-difluoro-5-phényl-1-[[2-(triméthylsilyl)éthoxy]méthyl]-1H-indazole-3-amine dans 8 ml de chlorure de méthylène, sont ajoutés successivement 131 µl de pyridine et 154µl de chloroformiate d'éthyle. Après 75 mn la réaction est terminée. Après hydrolyse, extraction et évaporation, on obtient 840 mg de (6,7-difluoro-5-phenyl-1H-indazol-3-yl)-carbamic acid ethyl ester brut.

### Etape2

A 161 mg de de (6,7-difluoro-5-phenyl-1H-indazol-3-yl)-carbamic acid ethyl ester brut.
dans 2.5 ml de trifluorotoluene, on ajoute 184mg de piperidine et l'on conduit la réaction sous micro-ondes 20 mn à 200°C. Après purification par LC/MS préparative (acétonitrile/tampon pH9) on obtient 80 mg de Piperidine-1-carboxylic acid (6,7-difluoro-5-phenyl-1-[[2-(triméthylsilyl)éthoxy]méthyl]-1H-indazol-3-yl)-amide

### Etape3

80 mg de Piperidine-1-carboxylic acid (6,7-difluoro-5-phenyl-1-[[2-(triméthylsilyl)éthoxy]méthyl]-1H-indazol-3-yl)-amide dans 2.5 ml de methanol sont traités par 0.82 ml d'HCl 2N 1h au reflux. Après évaporation et purification par LC/MS préparative (acétonitrile/tampon pH9) on obtient 11 mg de Piperidine-1-carboxylic acid (6,7-difluoro-5-phenyl-1H-indazol-3-yl)-amide.
Spectre de masse : temps de rétention 3.99 ; 357 = [M+H]⁺
Spectre de R.M.N. ¹H (300 MHz, (DMSO-d6, δ en ppm) : 1.50 (m,4H); 1.58 (m,2H) ; 3.45 (m,4H) ; 7.42 (m, 1H) ; 7.51 (m,5H) ; 9.16 (s,1H) ; 13.20 (s1,1H)

### Exemple 2 : Pyrrolidine-1-carboxylic acid (6,7-difluoro-5-phenyl-1H-indazol-3-yl)-amide

### Etape 1

A 161 mg de (6,7,difluoro-5-phenyl-1H-indazol-3-yl)-carbamic acid ethyl ester dans 2.5 ml de trifluototoluene, on ajoute 154 mg de pyrrolidine et l'on conduit la réaction sous micro-ondes 20 mn à 200°C.On purifie sur colonne de silice et on obtient 75mg de pyrrolidine-1-carboxylic acid (6,7-difluoro-5-phenyl-1-[[2-(triméthylsilyl)éthoxy]méthyl]-1H-indazol-3-yl)-amide.

### Etape 2

75 mg de Pyrrolidine-1-carboxylic acid (6,7-difluoro-5-phenyl-1-[[2-(triméthylsilyl)éthoxy]méthyl]-1H-indazol-3-yl)-amide dans 3ml de methanol sont traités par 0.82 ml d'HCl 2N lh au reflux. Après évaporation et purification par LC/MS préparative (acétonitrile/tampon pH9) on obtient 36mg de Pyrrolidine-1-carboxylic acid (6,7-difluoro-5-phenyl-1H-indazol-3-yl)-amide.
Spectre de masse : temps de rétention 3.72mn ; 343 = [M+H]⁺
Spectre de R.M.N. ¹H (300 MHz, (DMSO-d6, δ en ppm) : 1.86 (m,4H) ; 3.40 (m,4H) ; 7.42 (m,1H) ; de 7.45 à 7.54 (m,4H) ; 7.63 (dl, J= 7 Hz, 1H) ; 8.84 (s,1H) ; 13.20 (sl, 1H)

### Exemple 3 : fait selon l'exemple 2 à partir de la 3-(4-methyl-piperazin-1-yl)-propyl amine; on obtient le 1-(6,7-Difluoro-5-phenyl-1H-indazol-3-yl)-3-[3-(4-methyl-piperazin-1-yl)-propyl]-urea.

Spectre de R.M.N. ¹H (300 MHz, (DMSO-d6, δ en ppm) : 1.92 (m,2H) ; 2.82 (s,3H) ; de 3.01 à 3.75 (m, partiellement masqués, 12 H) ; 7.43 (m,1H) ; de 7.47 à 7.56 (m,4H) ; 7.71 (t,J=7Hz,1H) ; 8.05 (dd,J=1.5 - 7 Hz; 1H) ; 9.61 (s,1H)
Spectre de masse : temps de rétention 2.57mn ; 429 = [M+H]⁺

Les compositions pharmaceutiques selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

L'invention a pour objet les composés et leur utilisation d'aminoindazoles de formule (I) et leurs sels pharmaceutiquement acceptables pour la préparation de compositions pharmaceutiques destinées à prévenir et traiter les maladies pouvant résulter d'une activité anormale de kinases comme par exemple celles impliquées dans les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer, Comme activité anormale de kinase on peut citer par exemple celle de la PI3K, AkT, GSK3béta, des CDK's ...

En thérapeutique humaine, les composés selon l'invention sont particulièrement utiles pour le traitement et/ou la prévention des maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athéroselérotiques, le syndrome des ovaires polycystiques, le syndrome X, l'immunodéficience et le cancer.

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :

| | |
|---|---|
| - Composé de formule (I)..... | 50 mg |
| - Cellulose..... | 18 mg |
| - Lactose..... | 55 mg |
| - Silice colloïdale..... | 1 mg |
| - Carboxyméthylamidon sodique..... | 10 mg |
| - Talc..... | 10 mg |
| - Stéarate de magnésium..... | 1 mg |

### EXEMPLE B

On prépare selon la technique habituelle des comprimés dosés à 50 mg de produit actif ayant la composition suivante :

| | |
|---|---|
| - Composé de formule (I)..... | 50 mg |
| - Lactose..... | 104 mg |
| - Cellulose...... | 40 mg |
| - Polyvidone..... | 10 mg |
| - Carboxyméthylamidon sodique...... | 22 mg |
| - Talc..... | 10 mg |
| - Stéarate de magnésium..... | 2 mg |
| - Silice colloïdale..... | 2 mg |
| - Mélange d'hydroxyméthylcellulose, glycérine, oxyde de | |
| titane (72-3,5-24,5) q.s.p. 1 comprimé pelliculé terminé à 245 mg | |

### EXEMPLE C

On prépare une solution injectable contenant 10 mg de produit actif ayant la composition suivante :

| | |
|---|---|
| - Composé de formule (I)..... | 10 mg |
| - Acide benzoïque..... | 80 mg |
| - Alcool benzylique..... | 0,06 ml |
| - Benzoate de sodium..... | 80 mg |
| - Ethanol à 95 %..... | 0,4 ml |
| - Hydroxyde de sodium..... | 24 mg |
| - Propylène glyool..... | 1,6 ml |
| - Eau..... q.s.p. | 4 ml |

Est également décrite une méthode de prévention et de traitement des maladies dans lesquelles une phosphorylation de la protéine Tau est impliquée par administration d'un comparé de formule (I) et ses sels pharmaceutiquement acceptables.

## Revendications

1. Composés de formule (I) dans laquelle : 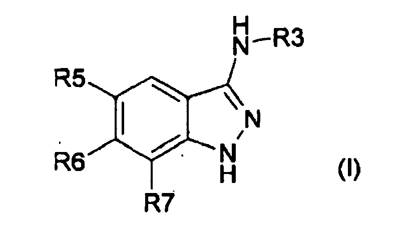
R3 est un radical (1-6C)alkyle, aryle(1-6C)alkyle, hétéroaryle(1-6C)alkyle, hétérocycloalkyle, cycloalkyle, adamantyle, polycycloalkyles, alkényle, alkynyle, CONR1R2, CSNR1R2, COOR1, SO2R1, C(=NH)R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, hétérocycle, formyle, trifluorométhyle, trifluorométhylsulfanyle, trifluorométhoxy, (1-6C)alkyle ;
R5, R6, sont indépendamment l'un de l'autre choisis parmi les radicaux suivant halogène, CN, NO2, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8, trifluorométhyle, trifluorométhoxy, (1-6C)alkyle, (1-6C)alcoxy, aryle, aryle(1-6C)alkyle, hétéroaryle, hétéroaryle(1-6C)alkyle, hétérocycle, cycloalkyle, alkényle, alkynyle, adamantyle, polycycloalkyles ; ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy, (1-6C)alkyle;
R7 est un halogène, méthyle, cyclopropyle, CN, OH, méthoxy, trifluorométhyle, éthylényle, acétylényle, trifluorométhoxy, NO2, NH2, NMe2
R1, R2, R8, R9, R10, R11 sont indépendamment l'un de l'autre un hydrogène, (1-6C)alkyle, aryle, alkényle, alkynyl, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ;
R1 et R2 ou R8 et R9 ou R10 et R11 peuvent former un cycle à 5 ou 6 chaînons ayant ou non un hétéroatome tel que O, S, N ;
leurs racémiques, énantiomères, diastéréoisomères et leurs mélanges, leurs tautomères et leurs sels pharmaceutiquement acceptables.

2. Composés de formule (I) dans laquelle : 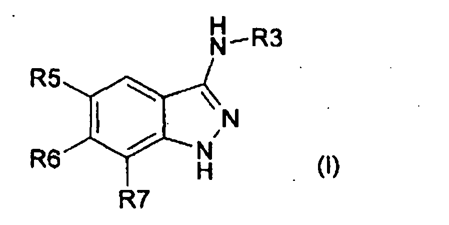
R3 est un radical (1-6C)alkyle, aryle(1-6C)alkyle, hétéroaryle(1-6C)alkyle, hétérocycloalkyle, cycloalkyle, adamantyle, polycycloalkyles, alkényle, alkynyle, CONR1R2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle;
R6, R7 sont indépendamment l'un de l'autre un halogène, méthyle, cyclopropyle, CN, OH, méthoxy, trifluorométhyle, éthylényle, acétylényle, trifluorométhoxy, N02, NH2, NMe2
R1, R2 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ;
R1 et R2 peuvent former un cycle à 5 ou 6 chaînons ayant ou non un hétéroatome tel que O, S, N ;
leurs racémiques, énantiomères, diastéréoisomères et leurs mélanges, leurs tautomères et leurs sels pharmaceutiquement acceptables.

3. Composé selon la revendication 1 **caractérisé par le fait qu'**il est choisi parmi :
N-(bicyclo[2,2,1]hept-5-en-2ylmethyl)-6-chloro-7-fluoro-5-phenyl-1H-indazol-3-amine
6-chloro-7-fluoro-N-(3,3-dimethylbutyl)-5-phenyl-1H-indazol-3-amine
6-chloro-7-fluoro-N-(3-phenylpropyl)-5-phenyl-1H-indazol-3-amine
6-chloro-7-fluoro-N-(cyclopropylmethyl)-5-phenyl-1H-indazol-3-amine
6-chloro-7-fluoro-N-(cyclopentylmethyl)-5-phenyl-1H-indazol-3-amine
6-chloro-7-fluoro-N-[3-(methylthio)propyl]-5-phenyl-1H-indazol-3-amine
6-chloro-7-fluoro-N-(phenylethyl)-5-phenyl-1H-indazol-3-amine
6-chloro-7-fluoro-N-(cyclohexylmethyl)-5-phenyl-1H-indazol-3-amine
6-chloro-7-fluoro-N-propyl-5-phenyl-1H-indazol-3-amine
6-chloro-7-fluoro-N-(2,2,3,3,4,4,4-heptafluorobutyl)-5-phenyl-1H-indazol-3-amine, hydrate
6-chloro-7-fluoro-N-(4,4,4-trifluorobutyl)-5-phenyl-1H-indazol-3-amine
6-chloro-7-fluoro-N-[(4-methoxyphenyl)methyl]-5-phenyl-1H-indazol-3-amine
6-chloro-7-fluoro-N-(phenylmethyl)-5-phenyl-1H-indazol-3-amine
6-chloro-7-fluoro-N-[(4-cyanophenyl)methyl]-5-phenyl-1H-indazol-3-amine
N-[(4-chlorophenyl)methyl]-6-chloro-7-fluoro-5-phenyl-1H-indazol-3-amine
6-chloro-7-fluoro-N-[(3-methoxyphenyl)methyl]-5-phenyl-1H-indazol-3-amine
6-chloro-7-fluoro-N-[[4-(trifluoromethoxy)phenyl]methyl]-5-phenyl-1H-indazol-3-amine
N-[4-[[[6-chloro-7-fluoro-5-phenyl-1H-indazol-3-yl]amino]methyl]phenyl]-acetamide
6-chloro-7-fluoro-N-[(3,5-dichlorophenyl)methyl]-5-phenyl-1H-indazol-3-amine
6-chloro-7-fluoro-5-phenyl-N-[[4-(trifluoromethyl)phenyl]methyl]-1H-indazol-3-amine
6-chloro-7-fluoro-N-[(4-fluorophenyl)methyl]-5-phenyl-1H-indazol-3-amine
6-chloro-7-fluoro-N-[3-(4-methylphenoxy)phenylmethyl]-5-phenyl-1H-indazol-3-amine
N-(2,2,3,3,4,4,4-heptafluorobutyl]-6-chloro-7-fluoro-5-phenyl-1H-indazol-3-amine
6-chloro-7-fluoro-5-phenyl-N-[[3,5-bis(trifluoromethyl)phenyl]methyl]-1H-indazol-3-amine
6-chloro-7-fluoro-5-phenyl-N-[[3-(trifluoromethyl)phenyl]methyl]-1H-indazol-3-amine
6-chloro-7-fluoro-N-[(6-methoxy-2-naphthalenyl)methyl]-5-phenyl-1H-indazol-3-amine
6-chloro-7-fluoro-N-[(pentafluorophenyl)methyl]-5-phenyl-1H-indazol-3-amine
6-chloro-7-fluoro-N-[[4-(methylthio)phenyl]methyl]-5-phenyl-1H-indazol-3-amine
N-[(4-chloro-3-fluorophenyl)methyl]-6-chloro-7-fluoro-5-phenyl-1H-indazol-3-amine
6-chloro-7-fluoro-5-phenyl-N-(3,3,3-trifluoropropyl)-1H-indazol-3-amine
6-chloro-7-fluoro-5-phenyl-N-(3-thienylmethyl)-1H-indazol-3-amine
N-(bicyclo[2,2,1]hept-5-en-2ylmethyl)-6-chloro-7-fluoro-5-phenyl-1H-indazol-3-amine
N-([1,1'-biphenyl]-4-ylmethyl)-6-chloro-7-fluoro-5-phenyl-1H-indazol-3-amine
6-chloro-7-fluoro-N-[[4-(dimethylamino)phenyl]methyl]-5-phenyl-1H-indazol-3-amine
N-([2,2'-bithiophen]-5-ylmethyl)-6-chloro-7-fluoro-5-phenyl-1 H-indazol-3-amine
6-chloro-7-fluoro-5-phenyl-N-[[1-(phenylmethyl)-1H-imidazol-2-yl]methyl]- 1H-indazol-3-amine
6-chloro-7-fluoro-N-[[1-methyl-1H-imidazol-2-yl]methyl]-5-phenyl- 1H-indazol-3-amine
6-chloro-7-fluoro-N-[(1-methyl-1H-indol-3-yl)methyl]-5-phenyl-1H-indazol-3-amine
6-chloro-7-fluoro-N-[(5-methyl-2-furanyl)methyl]-5-phenyl-1H-indazol-3-amine
6-chloro-7-fluoro-5-phenyl-N-(1H-pyrrol-2-ylmethyl)-1H-indazol-3-amine
6-chloro-7-fluoro-5-phenyl-N-(1H-imidazol-2-yl)methyl]-1H-indazol-3-amine
6-chloro-7-fluoro-5-phenyl-N-(1H-imidazol-4-yl)methyl]-1H-indazol-3-amine
6-chloro-7-fluoro-5-phenyl-N-(1H-pyrazol-3-ylmethyl)-1H-indazol-3-amine
6-chloro-7-fluoro-N-[[2-methyl-1H-imidazol-4-yl]methyl]-5-phenyl-1H-indazol-3-amine
6-chloro-7-fluoro-N-[(3,5-dimethyl-1-phenyl-1H-pyrazol-4-yl)methyl]-5-phenyl-1H-indazol-3-amine
6-chloro-7-fluoro-5-phenyl-N-[[2-phenyl-1H-imidazol-4-yl]methyl]-1H-indazol-3-amine
6-chloro-7-fluoro-N-[[5-(4-chlorophenyl)-2-furanyl]methyl]-5-phenyl-1H-indazol-3-amine
6-chloro-7-fluoro-5-phenyl-N-[(1-methyl-1H-pyrrol-2-yl)methyl]-1H-indazol-3-amine
4-[5-[[[6-chloro-7-fluoro-5-phenyl-1H-indazol-3-yl]amino]methyl]-2-furanyl]-Benzenesulfonamide
6-chloro-7-fluoro-5-phenyl-N-(3-thienylmethyl)-1H-indazol-3-amine
6-chloro-7-fluoro-5-phenyl-N-[[2-phenyl-1H-imidazol-4-yl]methyl]-1H-indazol-3-amine
2-[([6-chloro-7-fluoro-5-phenyl-1H-indazol-3-yl]amino]methyl]-5-(methylthio)-1H-imidazole-4-carboxylate d'éthyle
6-chloro-7-fluoro-5-phenyl-N-[[5-[4-(trifluoromethyl)phenyl]-2-furanyl]methyl]-1H-indazol-3-amine
6-chloro-7-fluoro-5-phenyl-N-[2-(1-piperidinyl)ethyl]-1H-indazol-3-amine
6-chloro-7-fluoro-N-[2-(4-morpholinyl)ethyl]-5-phenyl-1H-indazol-3-amine
N-(6-chloro-7-fluoro-5-phenyl-1H-indazol-3-yl)-N'-(3,5-dichlorophenyl)- Urée
N-(6-chloro-7-fluoro-5-phenyl-1H-indazol-3-yl)-N'-(2-propenyl)- Urée
N-(6-chloro-7-fluoro-5-phenyl-1H-indazol-3-yl)-N'-(phenylmethyl)- Urée
N-(6-chloro-7-fluoro-5-phenyl-1H-indazol-3-yl)-N'-4-(phenoxyphenyl)- Urée
N-(6-chloro-7-fluoro-5-phenyl-1H-indazol-3-yl)-N'-[(4-methoxyphenyl)methyl]-Urée
N-(6-chloro-7-fluoro-5=phenyl-1H-indazol-3-yl)-N'-[4-(trifluoromethyl)phenyl]- Urée
N-(6-chloro-7-fluoro-5-phenyl-1H-indazol-3-yl)-N'-{4-methoxyphenyl)- Urée
N-(6-chloro-7-fluoro-5-phenyl-1H-indazol-3-yl)-N'-cyclohexyl-Urée
N-(6-chloro-7-fluoro-5-phenyl-1H-indazol-3-yl)-N'-propyl- Urée
N-(6-chloro-7-fluoro-5-phenyl-1H-indazol-3-yl)-N'-(4-chlorophenyl)- Urée
N-(6-chloro-7-fluoro-5-phenyl-1H-indazol-3-yl)-N'-(4-fluorophenyl)- Urée
N-[6-chloro-7-fluoro-5-phenyl-1H-indazol-3-yl]-N'-tricyclo[3.3.1.13,7]dec-1-yl-Urée
N-(6-chloro-7-fluoro-5-phenyl-1H-indazol-3-yl)-N'-(4-methylphenyl)- Urée
N-(6-chloro-7-fluoro-5-phenyl-1H-indazol-3-yl)-Urée
N-(6-chloro-7-methyl-5-phenyl-1H-indazol-3-yl)-Urée
N-(6-chloro-7-cyano-5-phenyl-1H-indazol-3-yl)-Urée
N-(6-chloro-7-cyclopropyl-5-phenyl-1H-indazol-3-yl)-Urée
N-(6-chloro-7-hydroxy-5-phenyl-1H-indazol-3-yl)-Urée
N-(6-chloro-7-methoxy-5-phenyl-1H-indazol-3-yl)-Urée
N-(6-chloro-7-trifluoromethyl-5-phenyl-1H-indazol-3-yl)-Urée
N-(6-chloro-7-trifluoromethoxy-5-phenyl-1H-indazol-3-yl)-Urée
N-(6-chloro-7-nitro-5-phenyl-1H-indazol-3-yl)-Uréé
N-(6-chloro-7-amino-5-phenyl-1H-indazol-3-yl)-Urée
N-(6-chloro-7-dimethylamino-5-phenyi-1H-indazol-3-yl)-Urée
N-(6-chloro-7-ethynyl-5-phenyl-1H-indazol-3-yl)-Urée
N-[6-chloro-7-fluoro-5-phenyl-1H-indazol-3-yl]-4-methyl- Benzenesulfonamide
N-[6-chloro-7-fluoro-5-phenyl-1H-indazol-3-yl]-Methanesulfonamide
N-[6-chloro-7-fluoro-5-phenyl-1H-indazol-3-yl]-2-propanesulfonamide
N-[6-chloro-7-fluoro-5-phenyl-1H-indazol-3-yl]-2,2,2-trifluoro-ethanesulfonamide
N-[6-chloro-7- fluoro-5-phenyl-1H-indazol- 3-yl]-2-thiophenesulfonamide
N-[6-chloro-7-fluoro-5-phenyl-1H-indazol-3-yl]- Benzenesulfonamide
N-[6-chloro-7-fluoro-5-phenyl-1H-indazol-3-yl]-4-(trifluoromethyl)-Benzenesulfonamide
N-[6-chloro-7-fluoro-5-phenyl-1H-indazol-3-yl]-5-(3-isoxazolyl)-2-thiophenesulfonamide
N-[6-chloro-7-fluoro-5-phenyl-1H-indazol-3-yl]-4-fluoro-Benzenesulfonamide
N-(6-chloro-7-fluoro-5-phenyl-1H-indazol-3-yl]-4-methoxy-Benzenesulfonamide
N-[6-chloro-7-fluoro-5-phenyl-1H-indazol-3-yl]-Benzenemethanesulfonamide
N-[6-chloro-7-fluoro-5-phenyl-1H-indazol-3-yl]-1-methyl-1H-imidazole-4-sulfonamide
N-[6-chloro-7-fluoro-5-phenyl-1H-indazol-3-yl]-4-(1,1-dimethylethyl)-Benzenesulfonamide
N-[4-([(6-ehloro-7-fluoro-5-phenyl-1H-indazol-3-yl)amino]sulfonyl]phenyl]-Acetamide
N-[6-chloro-7-ftuoro-5-phcnyl-1H-indazol-3-yl]-4-methyl-Benzenemethanesulfonamide
6-chloro-7-fluoro-N-(pentafluorophenyl)-5-phenyl-1H-indazol-3-amine
6-chloro-7-fluoro-N-(3,4-difluorophenyl)-5-phenyl-1H-indazol-3-amine
6-chloro-7-fluoro-5-phenyl-N-(2, 3,5,6-tetrafluorophenyl)-1H-indazol-3-aminé
6-chloro-7-fluoro-5-phehyl-N-(2,4,6-trifluorophenyl)-1H-indazol-3-amine
6-chloro-7-fluoro-N-(4-fluorophenyl)-5-phenyl-1H-indazol-3-amine
6-chloro-7-fluoro-N-[3-(trifluoromethyl)phenyl]-5-phenyl-1H-indazol-3-amine
6-chloro-7-fluoro-N-[4-(trifluoromethyl)phenyl]-5-phenyl-1H-indazol-3-amine
6-chloro-7-fluoro-N-[3-fluoro-5-(trifluoromethyl)phenyl]-5-phenyl-1H-indazol-3-amine
6-chloro-7-fluoro-N-(4-nitrophenyl)-5-phenyl-1H-indazol-3-amine
6-chloro-7-fluoro-N-(3-nitrophenyl)-5-phenyl-1H-indazol-3-amine
6-chloro-7-fluoro-N-(3-methoxyphenyl)-5-phenyl-1H-indazol-3-amine
6-chloro-7-fluoro-N-(4-methoxyphenyl)-5-phenyl-1H-indazol-3-amine
6-chloro-7-fluoro-N,5-diphenyl-1H-indazol-3-amine
6-ohloro-7-fluoro-N-(1-pyridinyl)-5-phenyl-1H-indazol-3-amine
6-chloro-7-fluoro-N-(2-pyridinyl)-5-phenyl-1H-indazol-3-amine
N-butyl-6-chloro-7-fluoro-5-phenyl-1H-indazol-3-amine N-(6-chloro-7-fluoro-5-phenyl-1H-indazol-3-yl)-N'-phenyl-Urée N-(6-chloro-7-fluoro-5-phényl-1H-indazol-3-yl)-3-méthoxy-benzènesulfonamide leur racémiques, énantiomères, diastéréoisomères et leurs mélanges, leurs tautomères et leurs sels pharmaceutiquement acceptables.

4. Composé selon l'une quelconque des revendications 1 à 2 **caractérisé par le fait qu'**il est choisi parmi :
Piperidine-1-carboxylic acid (6,7-difluoro-5-phenyl-1H-indazol-3-yl)-amide
Pyrrolidine-1-carboxylic acid (6,7-difluoro-5-phenyl-1H-indazol-3-yl)-amide
ses tautomères ainsi que leurs sels pharmaceutiquement acceptables.

5. Composé **caractérisé par le fait qu'**il est choisi parmi :
1-(6,7.Difluoro-5-phenyl-1H-indazol-3-yl)-3-[3-(4-methyl-piperhin-1-yl)-propyl]-urea
ses tautomères ainsi que leurs sels pharmaceutiquement acceptables.

6. Composé selon l'une quelconque des revendications 1 à 5 utilisé pour préparer un médicament.

7. Composition pharmaceutique **caractérisée par le fait qu'**elle comprend dans un milieu pharmaceutiquement acceptable, un composé défini selon l'une quelconque des revendications 1 à 5.

8. Médicament selon la revendication 6 **caractérisé par le fait qu'**il contient au moins un composé défini selon l'une quelconque des revendications 1 à 5 pour son application thérapeutique dans le traitement des maladies dans lesquelles une phosphorylation de la protéine Tau est observée.

9. Médicament selon la revendication 6 **caractérisé par le fait qu'**il contient au moins un composé défini selon l'une quelconque des revendications 1 à 5 pour son application thérapeutique dans le traitement des maladies neurodégénératives, les accidents cérébrovasculaires, les traumatismes crâniens et spinaux et neuropathies périphériques, l'obésité, les maladies du métabolisme, le diabètede type II, l'hypertension essentielle, les maladies cardiovasculaires athétosclérotiques, le syndrome des ovaires polycystiques, le syndrome X, l'imunodéficience et le cancer.

10. Médicament selon la revendication 9 **caractérisé par le fait que** la maladie neurodégérative est soit la maladie d'Alzheimer, de Parkinson, la démence frontopariétale, la dégénération corticobasale ou la maladie de Pick.

11. Procédé de préparation des composés de formule (I) tels que définis dans la revendication 1 et pour lequel R3 est (1-6C)alkyle, aryle(1-6C)alkyle, hétéroaryle(1-6C)alkyle, hétérocycloalkyle, cycloalkyle, polycycloalkyles, ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle et R1, R2, sont indépendamment l'un de l'autre un hydrogène, (1-6C)alkyle, aryle, alkényle, alkynyl, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ; à partir d'un dérivé de formule (I) où R3 est H, d'un dérivé R1CHO et du triacétoxyborohydrure de sodium dans du dichlorométhane et éventuellement transformation du produit obtenu en sel pharmaceutiquement acceptable.

12. Procédé de préparation des composés de formule (I) tels que définis dans la revendication 1 et pour lequel R3 est CONR1R2, CSNR1R2, R1, R2, sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy à partir de OCNR1 et d'un dérivé de formule (I) où R3 est H dans du tétrahydrofurane et éventuellement transformation du produit obtenu en sel pharmaceutiquement acceptable.

13. Procédé de préparation des composés de formule (I) tels que définis dans la revendication 1 et pour lequel R3 est SO2R1 et R1 est un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy à partir de d'un chlorure de sulfonyle R1SO2C1 et d'un dérivé de formule (I) où R3 est H dans du dichlorométhane en présence d'une base et éventuellement transformation du composé obtenu en sel pharmaceutiquement acceptable.

14. A titre de produits intermédiaires
N-(6,7-difluoro-1H-indazol-3-yl)-butanamide:
N-[6,7-difluoro-1-[[2-(triméthylsilyl)éthoxy]mèthyl]-1H-indazol-3-yl]-butanamide
N-[5-bromo-6,7-idifluoro-1-[[2-(triméthylsilyl)éthoxy]méthyl]-1H-indazol-3-yl]-butanamide
N-[6,7-difluoro.5-phényl-1-[[2-(trimélhylsilyl)éthoxy]méthyl]-1H-indazol-3-yl]-butanamide
6,7-difluoro-5-phenyl-1-[[2-(triméthylsilyl)éthoxy]méthyl]-1H-indazole-3-amine

## Claims

1. Compounds of formula (I): 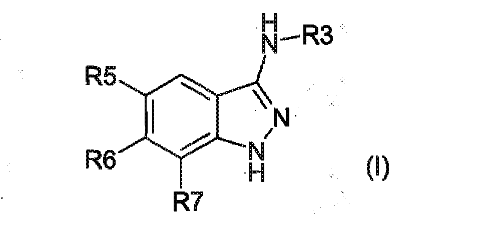 in which
R3 is a (1-6C)alkyl, aryl(1-6C) alkyl, heteroaryl(1-6C alkyl, heterocycloalkyl, cycloalkyl, adamantyl, polycycloalkyl, alkenyl, alkynyl, CONR1R2, CSNR1R2, COOR1, SO₂R1, C(=NH)R1 or C(=NH)NR1 radical; these radicals optionally being substituted by 1 or more substituents chosen from halogen, CN, NO₂, NH₂, OH, OR1, COOH, C(O)OR1, -O-C(O)R1, NR1R2, NHC(O)R1, C(O)NR1R2, SR1, S(O)R1 SO₂R1, NHSO₂R1, SO₂NR1R2, C(S)NR1R2, NHC(S)R1, -O-SO₂R1, -SO₂-O-R1, aryl, heteroaryl, heterocycle, formyl, trifluoromethyl, trifluoromethylsulfanyl, trifluoromethoxy or (1-6C)alkyl;
R5 and R6 are chosen, independently of one another, from the following radicals: halogen, CN, NO2, NH₂, OH, COOH, C(O)OR8, -O-C(O)R8, NR8R9, NHC(O)R8, C(O)NR8R9, NHC(S)R8, C(S)NR8R9, SR8, S(O)R8, SO₂R8, NHSO₂R8, SO₂NR8R9, -O-SO₂R8, -SO₂-O-R8; trifluoromethyl, trifluoromethoxy, (1-6C)alkyl, (1-6C)alkoxy, aryl, aryl(1-6C)alkyl, heteroaryl, heteroaryl(1-6C)alkyl, heterocycle, cycloalkyl, alkenyl, alkynyl, adamantyl, polycycloalkyls; these radicals optionally being substituted by 1 or more substituants chosen from halogen, CN, NO₂, NH₂, OH, OR10, OOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryl, heteroaryl, formyl, trifluoromethyl, trifluoromethoxy or (1-6C)alkyl;
R7 is a halogen, methyl, cyclopropyl, CN, OH, methoxy, trifluoromethyl, ethylenyl, acetylenyl, trifluoromethoxy, NO₂, NH₂ or NMe₂;
R1,R2, R8, R9, R10 and R11 are, independently of one another, a hydrogen, (1-6C)alkyl, aryl, alkenyl, alkynyl or heteroaryl, themselves optionally being substituted by 1 or more substituants chosen from halogen, (1-6C)alkyl, (1-6C)alkoxy, CN, NO₂, NH₂, OH, COOH, COOalkyl, CONH₂, formyl, trifluoromethyl or trifluoromethoxy;
R1 and R2 or R8 and R9 or R10 and R11 can form a 5- or 6-membered ring which may or may not have a heteroatom, such as O, S or N;
their racemates, enantiomers or diastereoisomers and their mixtures, their tautomers and their pharmaceutically acceptable salts.

2. Compounds: of formula (I) : 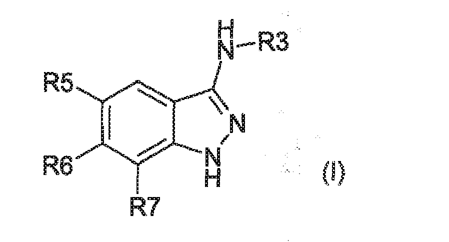 in which
R3 is a (1-6c)alkyl, aryl(1-6C)alkyl, heteroaryl(1-6C)alkyl, heterocycloalkyl, cycloalkyl, adamantyl, ppolycyaloalkyl, alkenyl, alkynyl, CONR1R2, CSNR1R2, COOR1, SO₂R1 or C(=NH)NR1 radical; these radicals optionally being substituted by 1 or more substituents chosen from halogen, CN, NO₂ ,NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, O-SO₂R1, -SO₂=O-R1, aryl, heteroaryl, formyl, oxo, trifluoromethyl, trifluoromethylsulfanyl, trifluoromethoxy or (1-6C)alkyl;
R5 is an aryl;
R6 and R7 are, independently of one another, a halogen, methyl, cyclopropyl, CN, OH, methoxy, trifluoromethyl, ethylenyl, acetylenyl, trifluoromethoxy, NO₂, NH₂ or NMe₂;
R1 and R2 are, independently of one another, a hydrogen, (1-6C)alkyl, aryl, alkynyl, alkynyl or heteroaryl, themselves optionally being substituted by 1 or more, substituents chosen from halogen, (1-6C)alkyl, (1-6C)alkoxy, CN, NO₂, NH₂, OH, COOH, COOalkyl, CONH₂, formyl, oxo, trifluoromethyl or trifluoromethoxy;
R1 and R2 can form a 5- or 6-membered ring which may or may not have a heteroatom, such as O, S or N; their racemates, enantiomers or diastereoisomers and their mixtures, their tautomers and their pharmaceutically acceptable salts.

3. Compound according to Claim 1, **characterized in that** it is chosen from:
N-(bicyclo[2.2.1]hept-5-en-2-ylmethyl)-6-chloro-7-fluoro-5-phenyl-1H-indazol-3-amine
6-chloro-7-fluoro-N-(3,3-dimethylbutyl)-5-phenyl-1H-indazol-3-amine
6-chloro-7-fluoro-N-(3-phenylpropyl) -5-phenyl-1H-indazol-3-amine
6-chloro-7-fluoro-N-(cyclopropylmethyl)-5-phenyl-1H-indazol-3-amine
6-chloro-7-fluoro-N-(cyclopentylmethyl)-5-phenyl-1H-indazol-3-amine
6-chloro-7-fluoro-N-[3-(methylthio)propyl]-5-phenyl-1H-indazol-3-amine
6-chloro-7-fluoro-N-(phenylethyl)-5-phenyl-1H-indazol-3-amine
6-chloro-7-fluoro-N-(cyclohexylmethyl)-5-phenyl-1H-indazol-3-amine
6-chloro-7-fluoro-N-propyl-5-phenyl-1H-indazol-3-amine 6-chloro-7-fluoro-N-(2,2,3,3,4,4,4-heptafluorobutyl)-5-phenyl-1H-indazol-3-amine hydrate
6-chloro-7-fluoro-N-(4,4,4-trifluorobutyl)-5-phenyl-1H-indazol-3-amine
6-chloro-7-fluoro-N-[(4-methoxyphenyl)methyl]-5-phenyl-1H-indazol-3-amine
6-chloro-7-fluoro-N-(phenylmethyl)-5-phenyl-1H-indazol-3-amine
6-chloro-7-fluoro-N-[(4-cyanophenyl)methyl]-5-phenyl-1H-indazol-3-amine
N-[(4-chlorophenyl)methyll-6-chloro-7-fluoro-5-phenyl-1H-indazol-3-amin.e
6-chloro-7-fluoro-N-[(3-methoxyphenyl)methyl]-5-phenyl-1H-idazol-3-amine
6-chloro-7-fluoro-N-[[4-(trifluoromethoxy)phenyl]methyl]-5-phenyl-1H-indazol-3-amine
N-[4-[[[6-chloro-7-fluoro-5-phenyl-1H-indazol-3-yl]amino]methyl]phenyl]acetamide
6-chloro-7-fluoro-N-[(3,5-dichlorophenyl)methyl]-5-phenyl-1H-indazol-3-amine
6'-chloro-7-fluoro-5-phenyl-N-[[4-(trifluoromethyl)phenyl]methyl]-1H-indazol-3-amine
6-chloro-7-fluoro-N-[(4-fluorophenyl)methyl]-5-phenyl-1H-indazol-3'-amine
6-chloro-7-fluoro-N-[3-(4-methylphenoxy)phenylmethyl]-5-phenyl-1H-indazol-3-amine
N-(2-,2,3,3,4,4,4-heptafluorobutyl)-6-chloro-7-fluoro-5-phenyl-1H-indazol-3-amine
6-chloro-7-fluoro-5-phenyl-N-[[3,5-bis(trifluoromethyl)phenyl]methyl]-1H-indazol-3-amine
6-chloro-7-fluoro-5-phenyl-N-[[3-(trifluoromethyl)phenyl]methyl]-1H-indazol-3-amine
6-chloro-7-fluoro-N-[(6-methoxy-2-naphthyl)methyl]-5-phenyl-1H-indazol-3-amine
6-hloro-7-fluoro-N-(pentafluorophenyl)methyl]-5-phenyl-1H-indazol-3-amine
6-chloro-7-fluoro-N-[[4-(methylthio)phenyl]methyl]-5-phenyl-1H-indazol-3-amine
N-[(4-chloro-3-fluorophenyl)methyl]-6-chloro-7-fluoro-5-phenyl-1H-indazol-3-amine
6-chloro-7-fluoro-5-phenyl-N-(3,3,3-trifluoropropyl)-1H-indazol-3-amine
6-chloro-7-fluoro-5-phenyl-N-(3-thienylmethyl)-1H-indazol-3-amine
N-(bicyclo[2.2.1]hept-5-en-2-ylmethyl)-6-chloro-7-fluoro-5-phepyl-1H-indazol-3-amine
N-(1,1'-biphenyl-4-ylmethyl)-6-chloro-7-fluoro-5-phenyl-1H-indazol-3-amine
6-Chloro-7-fluoro-N-[[4-dimethylamino)phenyl]methyl]-5-phenyl-1H-indazol-3-amine
N-(2,2'-bithiophen-5-ylmethyl)-6-chloro-7-fluoro-5-phenyl-1H-ihdazol-3-amine
6-chloro-7-fluoro-5-phenyl-N-[[1-(phenylmethyl)-1H-imidazol-2-yl]methyl]-1H-indazol-3-amine
6-chloro-7-fluoro-N-[[1-methyl-1H-imidazol-2-yl]methyl]-5-phenyl-1H-indazol-3-amine
6-chloro-7-fluoro-N-[(1-methyl-1H-indol-3-yl)methyl]-5-phenyl-1H-indazol-3-amine
6-chloro-7-fluoro-N-[(5-methyl-2-furanyl)methyl]-5-phenyl-1H-indazol-3-amine
6-chloro-7-fluoro-5-phenyl-N-(1H-pyrrol-2-ylmethyl)-1H-indazol-3-amine
6-phloro-7-fluoro-5-phenyl-N-(1H-imidazol-2-yl)methyl]-1H-indazol-3-amine
6-chloro-7-fluoro-5-phenyl-N-[(1H-imidazol-4-yl)methyl]-1H-indazol-3-amine
6-chloro-7-fluoro-5-phenyl-N-(1H-pyrazol-3-ylmethyl)-1H-indazol-3-amine
6-chloro-7-fluoro-N-[[2-methyl-1H-imidazol-4-yl] methyl-5-phenyl-1H-indazol-3-amine
6-chloro-7-fluoro-N-[(3,5-dimethyl-1-phenyl-1H-pyrazol-4-yl)methyl]-5-phenyl-1H-indazol-3-amine
6-chloro-7-fluoro-5-phenyl-N-[[2-phenyl-1H-imidazol-4-yl]methyl]-1H-indazol-3-amine
6-chloro-7-fluoro-N-[[5-(4-chlorophenyl)-2-furanyl]methyl]-5-phenyl-1H-indazol-3-amine
6-chloro-7-fluoro-5-phenyl-N-[(1-methyl-1H-pyrrol-2-yl)methyl]-1H-indazol-3-amine
4-[5-[[[6-chloro-7-fluoro-5-phenyl-1H-indazol-3-ylamino]methyl]-2-furanyl]-benzenesulfonamide
6-chloro-7-fluoro-5-phenyl-N-(3-thienylmethyl)-1H-indazol-3-amine
6-chloro-7-fluoro-5-phenyl-N-[[2-phenyl-1H-imidazol-4-yl]methyl]-1H-indazol-3-amine
ethyl 2-[[16-chloro-7-fluoro-5-phenyl-1H-indazol-3-yl]laminolmethyl]-5-(methylthio)-1H-imidazole-4-carboxylate
6-chloro-7-fluoro-5-phenyl-N-[[5-[4-(trifluoromethyl)phenyl]-2-furanyl]methyl]-1H-indazol-3-amine
6-chloro-7-fluoro-5-phenyl-N-[2-(1-piperidinyl)ethyl]-1H-indazol-3-amine
6-chloro-7-fluoro-N-[2-(4-morpholinyl)ethyl]-5-phenyl-1H-indazol-3-amine
N-(6-chloro-7-fluoro-5-phenyl-1H-indazol-3-yl)-N'-(3,5-dichlorophenyl)urea
N-(6-chloro-7-fluoro-5-phenyl-1H-indazol-3-yl)-N'-(2-propenyl)urea
N-(8-chloro-7-fluoro-5-phenyl-1H-indazol-3-yl)-N'-(phehylmethyl)urea
N-(6-chloro-7-fluoro-5-phenyl-1H-indazol-3-yl)-N'-(4-phenoxyphenyl)urea
N-(6-chloro-7-fluoro-5-phenyl-1H-indazol-3-yl)-N'-(4-methoxyphenyl)methyl]urea
N-(6-chloro-7-fluoro-5-phenyl-1H-indazol-3-yl)-N'-[4-(trifluorotnethyl)phenyl]urea
N-(6-chloro-7-fluoro-5-phenyl-1H-indazol-3-yl)-N'-(4-methoxyphenyl)urea
N-(6-chloro-7-fluoro-5-phenyl-1H-indazol-3-yl)-N'-cyclohexylurea
N-(6-chloro-7-fluoro-5-phenyl-1H-indazol-3-yl)-N-propylurea
N-(6-chloro-7-fluoro-5-phenyl-1H-indazol-3-yl)-N'-(4-chlorophenyl)urea
N-(6-chloro-7-fluoro-5-phenyl-1H-indazol-3-yl)-N'-(4-fluorophenyl)urea
N-[6-chloro-7-fluoro-5-phenyl-1H-indazol-3-yl]-N'-(tricyclo[3.3.1.1^{3,7}]dec)-1-ylurea
N-(6-'chloro-7-fluoro-5-phenyl-1H-indazol-3-yl)-N'-(4-methylphenyl)urea
N-(6-chloro-7-fluoro-5-phenyl-1H-indazol-3-yl)urea
N-(6-chloro-7-methyl-5-phenyl-1H-indazol-3-yl)urea
N-(6-chloro-7-cyano-5-phenyl-1H-indazol-3-yl)urea
N-(6-chloro-7-cyclopropyl-5-phenyl-1H-indazol-3-yl)urea
N-(6-chloro-7-hydroxy-5-phenyl-1H-indazol-3-yl)urea N-(6-chloro-7-methoxy-5-phenyl-1H-indazol-3-yl)urea
N-(6-chloro-7-trifluoromethyl-5-phenyl-1H-indazol-3-yl)urea
N-(6-chloro-7-trifluoromethoxy-5-phenyl-1H-indazol-3-yl)urea
N-(6-chloro-7-nitro-5-phenyl-1H-indazol-3-yl)urea
N-(6-chloro-7-amino-5-phenyl-1H-indazol-3-yl)urea
N'-(6-chloro-7-dimethylamino-5-phenyl-1H-indazol-3-yl)urea
N-(6-chloro-7-ethynyl-5-phenyl-1H-indazol-3-yl)urea
N-[6-chloro-7-fluoro-5-phenyl-1H-indazol-3-yl]-4-methyl-benzene sulfonamide
N-[6-chloro-7-fluoro-5-phenyl-1H-indazol-3-yl]methanesulfonamide
N-[6-chloro-7-fluoro-5-phenyl-1H-indazol-3-yl]-2-propanesulfonamide
N-[6-chloro-7-fluoro-5-phenyl-1H-indazol-3-yl]-2,2,2-trifluoroethanesulfonamide
N-[6-chloro-7-fluoro-5-phenyl-1H-indazol-3-yl]-2-thiophenesulfonamide
N-[6-chloro-7-fluoro-5-phenyl-1H-indazol-3-yl]benzenesulfonamide
N-[6-chloro-7-fluoro-5-phenyl-1H-indazol-3-yl]-4-(trifluoromethyl)benzenesulfonamide
N-[6-chloro-7-fluoro-5-phenyl-1H-indazol-3-yl]-5-(3-isoxazolyl)-2-thiophenesulfonamide
N-[6-chloro-7-fluoro-5-phenyl-1H-indazol-3-yl]-4-fluorobenzenesulfonamide
N-[6-chloro-7-fluoro-5-phenyl-1H-indazol-3-yl]-4-methoxybenzenesulfonamide
N-[6-chloro-7-fluoro-5-phenyl-1H-indazol-3 yl]benzenemethanesulfonamide
N-[6-chloro-7-fluoro-5-phenyl-1H-indazol-3-yl]-1-methyl-1H-imidazole-4-sulfonamide
N-[6-chloro-7-fluoro-5-phenyl-1H-indazol-3-yl]-4-(1,1-dimethylethyl)benzenesulfonamide
N-[4-[[(6-chloro-7-fluoro-5-phenyl-1H-indazol-3-yl)amino]sulfonyl]phenyl]acetamide
N-[6-chloro-7-fluoro-5-phenyl-1H-indazol-3-yl]-4-methylbenzenemethanesulfonamide
6-chloro-7-fluoro-N-(pentafluorophenyl)-5-phenyl-1H-indazol-3-amine
6-chloro-7-fluoro-N-(3,4-difluorophenyl)-5-phenyl-1H-indazol-3-amine
6-chloro-7-fluoro-5-phenyl-N-(2,3,5,6-tetrafluorophenyl)-1H-indazol-3-amine
6-chloro-7-fluoro-5-phenyl-N-(2,4,6-trifluorophenyl)-1H-indazol-3-amine
6-chloro-7-fluoro-N-(4-fluorophenyl)-5-phenyl-1H-indazol-3-amine
6-chloro-7-fluoro-N-[3-(trifluoromethyl)phenyl]-5-phenyl-1H-indazol-3-amine
6-phloro-7-fluoro-N-[4-(trifluoromethyl)phenyl]-5-phenyl-1H-indazol-3-amine
6-chloro-7-fluoro-N-[3-fluoro-5-(trifluoromethyl)phenyl]-5-phenyl-1H-indazol-3-amine
6-chloro-7-fluoro-N-(4-nitrophenyl)-5-phenyl-1H-indazol-3-amine
6-chloro-7-fluoro-N-(3-nitrophenyl)-5-phenyl-1H-indazol-3-amine
6-chloro-7-fluoro-N-(3-methoxyphenyl)-5-phenyl-1H-indazol-3-amine
6-chloro-7-fluoro-N-(4-methoxyphenyl)-5-phenyl-1H-indazol-3-amine
6-chloro-7-fluoro-N,5-diphenyl-1H-indazol-3-amine
6-chloro-7-fluoro-N-(1-pyridinyl)-5-phenyl-1H-indazol-3-amine
6-chloro-7-fluoro-N- (2-pyridinyl) -5-phenyl-1H-indazol-3-amine
N-butyl-6-chloro-7-fluoro-5-phenyl-1H-indazol-3-amine
N-(6-chloro-7-fluoro-5-phenyl-1H-indazol-3-yl)-N'-phenylurea
N-(6-chloro-7-fluoro-5-phenyl-1H-indazol-3-yl)-3-methoxybenzenesulfonamide
their racemates, enantiomers or diastereoisomers and their mixtures, their tautomers and their pharmaceutically acceptable salts.

4. Compounds according to either one of Claims 1 and 2, **characterized in that** it is chosen from:
Piperidine-1-carboxylic acid (6,7-difluoro-5-phenyl-1H-indazol-3-yl)amide
Pyrrolidine-1-carboxylic acid (6,7-difluoro-5-phenyl-1H-indazol-3-yl)amide
their tautomers, and their pharmaceutically acceptable salts.

5. Compound, **characterized in that** it is chosen from:
1-(6,7-Difluoro-5-phenyl-1H-indazol-3-yl)-3-[3-(4-methylpiperazin-1-yl)propyl]urea
its tautomers, and their pharmaceutically acceptable salts.

6. Compound according to any one of Claims to 5, used to prepare a medicament.

7. Pharmaceutical composition, **characterized in that** it comprises, in a pharmaceutically acceptable medium, a compound defined according to any one of Claims 1 to 5.

8. Medicament according to Claim 6, **characterized in that** it comprises at least one compound defined according to any one of Claims 1 to 5 for its therapeutic application in the treatment of diseases in which a phosphorylation of the tau protein is observed.

9. Medicament according to Claim 6, **characterized in that** it comprises at least one compound defined according to any one of Claims 1 to 5 for its therapeutic application in the treatment of neurodegenerative diseases, strokes, cranial and spinal traumas and-peripheral neuropathies, obesity, metabolic disease, type II diabetes, essential hypertension, atherosclerotic cardiovascular diseases, polycystic ovaries syndrome, syndrome X, immunodeficiency and cancer.

10. Medicament according to Claim 9, **characterized in that** it the neurodegenerative disease is either Alzheimer's disease, Parkinson's disease, frontoparietal dementia, corticobasal degeneration or Pick's disease.

11. Process for the preparation of the compounds of formula (I) as define in Claim. 1 and for which R3 is (1-6C)alkyl, aryl(1-6C)alkyl, heteroaryl(1-6C)alkyl, heterocycloalkyl, cycloalkyl or polycycloalkyl, these radicals optionally being substituted by 1 or more substituents chosen from halogen, CN, N02, NH2, OH, OR1, COOH, C(O)OR1, -O-C(O)R1, NR1R2, NHC(O)R1, C(O)NR1R2, -SR1, S(O)R1, SO2R1, NHSO2R1, SO2NR1R2 C(S)NR1R2, NHC(S)R1, -O-SO₂R1, -SO2-O-R1, aryl, heteroaryl, formyl, oxo, trifluoromethyl, trifluoromethylsulfanyl, trifluoromethoxy or (1-6C)alkyl and R1, R2 are, independently of one another, a hydrogen, (1-6C)alkyl, aryl, alkenyl, alkynyl or heteroaryl, themselves optionally being substituted by 1 or more substituents chosen from halogen, (1-6C) alkyl, (1-6C)alkoxy, CN, N02, NH2,
OH, COOH, COOalkyl, CONH₂, formyl, oxo, trifluoromethyl or trifluoromethoxy; from a derivative of formula (I) where R3 is H, from a derivative of RICHO and from sodium triacetoxyborohydride in dichloromethane, and optionally for the conversion of the product obtained to a pharmaceutically acceptable salt.

12. Process for the preparation of the compounds of formula (I) as defined in Claim 1 and for which R3 is CONR1R2 or CSNR1R2 and R1 and R2 are, independently of one another_{,} a hydrogen, (1-6C)alkyl, aryl, alkenyl, alkynyl or heteroaryl, themselves optionally being substituted by 1 or more substituents chosen from halogen, (1-6C)alkyl, (1-6C)alkoxy, CN, NO₂, NH₂, OH, COOH, COOalkyl, CONH₂, formyl, oxo, trifluoromethyl or trifluoromethoxy, from OCNR1 and from a derivative of formula (I) where R3 is H in tetrahydrofuran, and optionally for the conversion of the product obtained to a pharmaceutically acceptable salt.

13. Process for the preparation of the compounds of formula (I) as defined in Claim 1 and for which R3 is SO₂R1 and R1 is a hydrogen, (1-6C)alkyl, aryl, alkenyl, alkynyl or heteroaryl, themselves optionally being substituted by 1 or more substituents chosen from halogen, (1-6C)alkyl, (1-6C)alkoxy, CN, NO₂, NH₂, OH, COOH, COOalkyl, CONH₂, formyl, oxo, trifluoromethyl or trifluoromethoxy, from a sulfonyl chloride R1SO₂Cl, and from a derivative of formula (I) where R3 is H and dichlorométhane in the presence of a base, and optionally for the conversion of the compound obtained to a pharmaceutically acceptable salt.

14. As intermediate products,
N-(6,7-difluoro-1H-indazol-3-yl)butanamide
N-[6,7-difluoro-1-[[2-(trimethylsilyl)ethoxy]methyl]-1H-indazol-3-yl]butanamide
N-[5-bromo-6,7-difluoro-1-[[2-(trimethylsilyl)ethoxy]methyl]-1H-indazol-3-yl]-butanamide
N-[6,7-difluoro-5-phenyl-1-[[2-(trimethylsilyl)ethoxylmethyl]-1H-indazol-3-yl]-butanamide
6,7-difluoro-5-phenyl-1-[[2-(trimethylsilyl)ethoxy]methyl]-1H-indazole-3-amine.

## Patentansprüche

1. Verbindungen der Formel (I) : 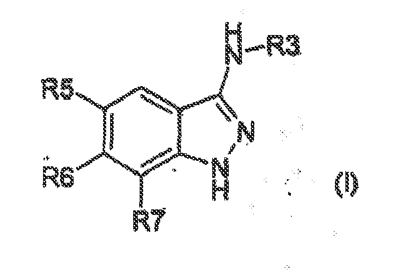 worin
R3 für einen (1-6C)-Alkyl-, Aryl-(1-6C)-alkyl-Heteroaryl-(1-6C)-alkyl-, Heterocycloalkyl-, Cycloalkyl-, Adamantyl-, Polycycloalkyl-, Alkenyl-, Alkenyl-, CONR1R2-, CSNR1R2-, COOR1-, SO₂R1-, C(=NH)R1- oder C(=NH)NR1-Rest steht; wobei diese Reste gegebenenfalls durch 1 oder mehrere unter Halogen, CN, NOₐ, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C (S) NR1R2 , NHC(S)R1, -O-SO₂R1, -SO₂-O-R1, Aryl, Heteroaryl, Heterocyclyl, Formyl, Trifluormethyl, Trifluormethylsulfanyl, Trifluormethoxy und (1-6C)-Alkyl ausgewählte Substituenten substituiert sind;
R5 und R6 unabhängig voneinander unter den folgende Resten ausgewählt sind: Halogen, CN, NO2, NH₂, OH, COOH, C(O)OR8, -O-C(O)R8, NR8R9, NHC(O)R8, C(O)NR8R9, NHC(S)R8, C(S)NR8R9, SR8, SS(O)R8, SO₂R8, NHSO₂R8, SO₂NR8R9, -O-SO₂R8, -SO₂-O-R8, Trifluormethyl, Trifluormethoxy, (1-6C)-Alkyl, (1-6C)-Alkoxy, Aryl, Aryl-(1-6C)-alkyl, Heteroaryl, Heteroaryl-(1-6C)-alkyl, Heterocyclyl, Cycloalkyl, Alkenyl, Alkinyl, Adamantyl und Polycycloalkyl; wobei diese Reste gegebenenfalls durch 1 oder mehrere unter Halogen, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, Aryl Heteroaryl, Formyl, Trifluormethyl, Trifluormethoxy und (1-6c)-Alkyl ausgewählte Substituenten substituiert sind;
R7 für ein Halogen, Methyl, cyclopropyl, CN, OH, Methoxy, Trifluormethyl, Ethylenyl, Acetylenyl, Trifluormethoxy, NO₂, NH₂ oder NMe₂ steht;
R1, R2, R8, R9, R10 und R11 unabhängig voneinander für Wasserstoff, (1-6C)-Alkyl, Aryl, Alkenyl, Alkinyl oder Heteroaryl, die selbst gegebenenfalls durch 1 oder mehrere unter Halogen, (1-6C)-Alkyl, (1-6C)-Alkoxy, CN, NO₂, NH₂, OH, COOH, COO-Alkyl, CONH₂, formyl, Trifluormethyl und Trifluormethoxy ausgewählte Substituenten substituiert sind, stechen;
R1 und R2 oder R8 und R9 oder R10 und R1 einen 5-oder 6-gliedrigen Ring, der gegebenenfalls ein Heteroatom wie O, S oder N aufweist, bilden können;
Racemate, Enantiomere und Diastereoisomere davon und Gemische davon, Tautomere davon und pharmazeutisch unbedenkliche Salze davon.

2. Verbindungen der Formel (I) : 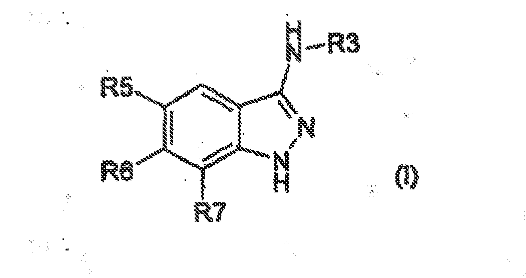 worin
R3 für einen (1-6C)-Alkyl-, Aryl-(1-6C)-alkyl-, Heteroaryl-(1-6C)-alkyl-, Heterocycloalkyl-, Cycloalkyl-, Adamantyl-, Polycycloalkyl-, Alkenyl-, Alkinyl-, CONR1R2-, CSNR1R2-, COOR1-, SO₂R1- oder C(=NH)NR1-Rest steht; wobei diese Reste gegebenenfalls durch 1 oder mehrere unter Halogen,
CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, Aryl, Heteroaryl, Formyl, Oxo, Trifluormethyl Trifluormethylsulfanyl, Trifluormethoxy und (1-6C)-Alkyl ausgewählte Substituenten substituiert sind;
R5 für Aryl steht;
R6 und R7 unabhängig voneinander für ein Halogen, Methyl, Cyclopropyl, CN, OH, Methoxy, Trifluorethyl, Ethylenyl, Acetylenyl, Trifluormethoxy, NO₂, NH₂ oder NMe₂ stehen;
R1 und R2 unabhängig voneinander für Wasserstoff, (1-6C)-Alkyl, Aryl, Alkenyl, Alkinyl oder Heteroaryl, die selbst gegebenenfalls durch 1 oder mehrere unter Halogen, (1-6C)-Alkyl, (1-6C)-Alkoxy, CN, NO₂, NH₂, OH, COOH, COO-Alkyl, CONH₂, ormyl, Oxo, Trifluormethyl und Trifluormethoxy ausgewählte Substituenten substituiert sind, stehen;
R1 und R2 einen 5- oder 6-gliedrigen Ring, der gegebenenfalls ein Heteroatom wie 0, S oder N aufweist, bilden können;
Racemate, Enantiomere und Diastereoisomere davon und Gemische davon, Tautomere davon und pharmazeutisch unbedenkliche Salze davon.

3. Verbindung nach Anspruch 1, **dadurch gekennzeichnet, daß** sie ausgewählt ist unter:
N-(Bicyclo[2.2.1]hept-5-en-2-ylmethyl)-6-chlor-7-fluor-5-phenyl-1H-indazol-3-amin
6-Chlor-7-fluor-N-(3,3-dimethylbutyl)-5-phenyl-1H-indazol-3-amin
6-Chlor-7-fluor-N-(3-phenylpropyl)-5-phenyl-1H-indazol-3-amin
6-chlor-7-fluor-N-(cycloprapylmethyl)-5-phenyl-1H-indazol-3-amin
6-Chlor-7-fluor-N-(cyclopentylmethyl)-5-phenyl-1H-indazol-3-amin
6-Chlor-7-fluor-N-[3-(methylthio)propyl]-5-phenyl-1H-indazol-3-amin
6-Chlor-7-fluor-N-(phenylethyl)-5-phenyl-1H-indazol-3-amin
6-Chlor-7-fluor-N-(cyclohexylmethyl)-5-phenyl-1H-indazol-3-amin
6-Chlor-7-fluor-N-propyl-5-phenyl-1H-indazol-3-amin
6-Chlor-7-fluor-N-(2,2,3,3,4,4,4-heptafluorbutyl)-5-phenyl-1H-indazol-3-amin-hydrat
6-Chlor-7-fluor-N-(4,4,4-trifluorbutyl)-5-phenyl-1H-indazol-3-amin
6-chlor-7-fluor-N-[(4-methoxyphenyl)methyl]-5-phenyl-1H-indazol-3-amin
6-Chlor-7-fluor-N-(phenylmethyl)-5-phenyl-1H-indazol-3-amin
6-Chlor-7-fluor-N-[(4-cyanophenyl)methyl]-5-phenyl-1H-indazol-3-amin
N-[(4-chlorphenyl)methyl]-6-chlor-7-fluor-5-phenyl-1H-indasol-3-amin
6-chlor-7-fluor-N-[(3-methoxyphenyl)methyl]-5-phenyl-1H-indazol-3-amin
6-Chlor-7-fluor-N-[[4-(trifluormethoxy)phenyl]-methyl]-5-phenyl-1H-indazol-3-amin
N-[-1-[[[6-Chlor-7-fluor-5-phenyl-1H-indazol-3-yl]amino]methyl]phenyl]acetamid
6-Chlor-7-fluor-N-[(3,5-dichlorphenyl)methyl]-5-phenyl-1H-indazol-3-amin
6-Chlor-7-fluor-5-phenyl-N-[[4-(trifluormethyl)-phenyl]methyl]-1H-indazol-3-amin
6-Chlor-7-fluor-N-[(4-fluorphenyl)methyl]-5-pphenyl-1H-indazol-3-amin
6-Chlor-7-fluor-N-[3-(4-methylphenoxy)phenylmethyl]-5-phenyl-1H-indazol-3-amin
N-(2,2,3,3,4,4,4-Heptafluorbutyl)-6-chlor-7-fluor-5-phenyl-1H-indazol-3-amin
-6-Chlor-7-fluor-5-phenyl-N-[[3,5-bis(trifluormethyl)phenyl]methyl]-1H-indazol-3-amin
6-Chlor-7-fluor-5-phenyl-N-[[3-(trifluormethyl)-phenyl]methyl]-1H-indazol-3-amin
6-Chlor-7-fluor-N-[(6-methoxy-2-naphthalinyl)methyl]-5-phenyl-3-H-indazol-3-amin
6-Chlor-7-fluor-N-[(pentafluorphenyl)methyl]-5-phenyl-1H-indazol-3-amin
6-Chlor-7-fluor-N-[[4-(methylthio)phenyl]methyl]-5-phenyl-1H-indazol-3-amin
N-[(4-Chlor-3-fluorphenyl)methyl]-6-chlor-7-fluor-5-phenyl-1H-indazol-3-amin
6-Chlor-7-fluor-5-phenyl-N-(3,3,3-trifluorpropyl)-1H-indazol-3-amin
6-Chlor-7-fluor-5-phenyl-N-(3-thienylmethyl)-1H-indazol-3-amin
N-(Bicyclo[2.2.1]hept-5-en-2-ylmethyl)-6-chlor-7-fluor-5-phenyl-1H-indazol-3-amin
N-([1,1'-Biphenyl]-4-ylmethl1)-6-chlor-7-fluor-5-phenyl-1H-indazol-3-amin
6-Chlor-7-fluor-N-[[4-(dimethylamino)phenyl]-methyl]-5-phenyl-1H-indazol-3-amin
N-([2,2'-Bithiophen]-5-ylmethyl)-6-chlor-7-fluor-5-phenyl-1H-indazol-3-amin
6-Chlor-7-fluor-5-phenyl-N-[[1-(phenylmethyl)-1H-imidazol-2-yl]methyl]-1H-indazol-3-amin
6-Chlor-7-fluor-N-[[1-methyl-1H-imidazol-2-yl]-methyl]-5-phenyl-1H-indazol-3-amin
6-Chlor-7-fluor-N-[(1-methyl-1H-indol-3-yl)-methyl]-5-phenyl-1H-indasol-3-amin.
6-Chlor-7-fluor-N-[(5-methyl-2-furanyl)methyl]-5-phenyl-1H-indazol-3-amin
6-Chlor-7-fluor-5-phenyl-N-(1H-pyrrol-2-ylmethyl)-1H-indazol-3-amin
6-Chlor-7-fluor-5-phenyl-N-[(1H-imidazol-2-yl)-methyl]-1H-indazol-3-amin
6-Chlor-7-fluor-5-phenyl-N-[(1H-imidazol-4-yl)-methyl]-1H-indazol-3-amin
-Chlor-7-fluor-5-phenyl-N-(1H-Pyrazol-3-yl-methyl)-1H-indasol-3-amin
6-Chlor-7-fluor-N-[[2-methyl-1H-imidazol-4-yl]-methyl]-5-phenyl-1H-indazol-3-amin
6-Chlor-7-fluor-N-[(3,5-dimethyl-1-phenyl-1H-pyrazol-4-yl)methyl]-5-phenyl-1H-indazol-3-amin
6-Chlor-7-fluor-5-phenyl-N-[[2-phenyl-1H-imidazol-4-yl]methyl]-1H-indazol-3-amin
6-Chlor-7-fluor-N-[[5-(4-chlorphenyl)-2-furanyl]-methyl]-B-phenyl-1H-indazol-3-amin
6-Chlor-7-fluor-5-phenyl-N-[(1-methyl-1H-pyrrol-2-yl)methyl]-1H-indazol-3-amin
4-[5-[[[6-Chlor-7-fluor-5-phenyl-1H-indazol-3-yl]-amino]methyl]-2-furanyl]-benzolsulfonamid
6-Chlor-7-fluor-5-phenyl-N-(3-thienylmethyl)-1H-indazol-3-amin
6-Chlor-7-fluor-5-phenyl-N-[[2-phenyl-1H-imidazol-4-yl]methyl]-1H-indazol-3-amin
2-[[[6-Chlor-7-fluor-5-phenyl-1H-indazol-3-yl]-amino]methyl]-5-(methylthio)-1H-imidazol-4-carbonsäureethylester
6-chlor-7-fluor-5-phenyl-N-[[5-14-(trifluormethyl)phenyl]-2-furanyl]methyl]-1H-indazol-3-amin
6-Chlor-7-fluor-5-phenyl-N-[2-(1-piperidinyl)-ethyl]-1H-indazol-3-amin
6-Chlor-7-fluor-N-[2-(4-morpholinyl)ethyl]-5-phenyl-1H-indazol-3-amin
N-(6-chlor-7-fluor-5-phenyl-1H-indazol-3-yl)-N-(3, 5-dichlorphenyl)harnstoff
N-(6-Chlor-7-fluor-5-phenyl-1H-indazol-3-yl)-N'-(2-propenyl)harnstoff
N-(6-Chlor-7-fluor-5-phenyl-1H-indazol-3-yl)-N'-(phenylmethyl)harnstoff
N-(6-Chlor-7-fluor-5-phenyl-1H-indazol-3-yl)-N'-(4-phenoxyphenyl)harnstoff
N-(6-chlor-7-fluor-5-phenyl-1H-indazol-3-yl)-N'-[(4-methoxyphenyl)methyl]harnstoff
N-(6-chior-7-fluor-5-phenyl-1H-indazol-3-yl)-N'-4-(trifluormethyl)phenyl]harxistoff
N-(6-Chlor-7-fluor-5-phenyl-1H-indazol-3-yl)-N'-(4-methoxyphenyl)harnstoff
N-(6-Chlor-7-fluor-5-phenyl-1H-indazol-3-yl)-N'-cyclohexylharnstoff
N-(6-Chlor-7-fluor-5-phenyl-1H-indazol-3-yl)-N'-propylharnstoff
N-(6-Chlor-7-fluor-5-phenyl-1H-indazol-3-yl)-N'-(4-chlorphenyl)harnstoff
N-(6-Chlor-7-fluor-5-phenyl-1H-indazol-3-yl)-N'-(4-fluorphenyl)harnstoff
N-[6-Chlor-7-fluor-5-phenyl-1H-indazol-3-yl]-N'-tricyclo[3.3.1.1^{3,7}]dec-1-ylharnstoff
N-(6-Chlor-7-fluor-5-phenyl-1h-indazol-3-yl)-N'-(4-methylphenyl)harnstoff
N-(6-Chlor-7-fluor-5-phenyl-1H-indazol-3-yl)-harnstoff
N-(6-Chlor-7-methyl-5-phenyl-1H-indazol-3-yl)-harnstoff
N-(6-Chlor-7-cyano-5-phenyl-1H-indazol-3-yl)-harnstoff
N-(6-Chlor-7-cyclopropyl-5-phenyl-1H-indazol-3-yl) harnstoff
N-(6-Chlor-7-hydroxy-5-phenyl-1H-indazol-3-yl)-hartstoff
N-(6-Chlor-7-methoxy-5-phenyl-1H-indasol-3-yl)-harnstoff
N-(6-Chlor-7-trifluormethyl-5-phenyl-1H-indazol-3-yl)harnstoff
N-(6-Chlor-7-trifluormethoxy-5-phenyl-1H-indazol-3-yl)harnstoff
N-(6-Chlor-7-nitro-5-phenyl-1H-indazol-3-yl)-harnstoff
N-(6-Chlor-7-amino-5-phenyl-1H-indazol-3-yl)-harnstoff
N-(6-Chlor-7-dimethylamino-5-phenyl-1H-indazol-3-yl)harnatoff
N-(6-Chlor-7-ethinyl-5-phenyl-1H-indazol-3-yl)-harnstoff
N-[6-Chlor-7-fluor-5-phenyl-1H-indazol-3-yl]-4-methylbenzolsulfonamid
N-[6-chlor-7-fluor-5-phenyl-1H-indazol-3-yl]-methansulfonamid
N-[6-Chlor-7-fluor-5-phenyl-1H-indazol-3-yl]-2-propansulfonamid
N-[6-chlor-7-fluor-5-phenyl-1H-indazol-3-yl]-2,2,2-trifluorethansulfonamid
N-[6-Chlor-7-fluor-5-phenyl-1H-indazol-3-yl]-2-thiophensulfonamid
N-[6-Chlor-7-fluor-5-phenyl-1H-indazol-3-yl]-benzolsulfonamid
N-[6-Chlor-7-fluor-5-phenyl-1H-indazol-3-yl]-4-(trifluormethyl)benzolsulfonamid
N-[6-Chlor-7-fluor-5-phenyl-1H-indazol-3-yl]-5-(3-isoxazolyl)-2-thiophensulfonamid
N-[6-Chlor-7-fluor-5-phenyl-1H-indazol-3-yl]-4-fluorbenzolsulfonamid
N-[6-Chlor-7-fluor-5-phenyl-1H-indazol-3-yl]-4-methoxybenzolsulfonamid
N-[6-Chlor-7-fluor-5-phenyl-1H-indazol-3-yl]benzolmethansulfonamid
N-[6-Chlor-7-fluor-5-phenyl-1H-indazol-3-yl]-1-methyl-1H-imidazol-4-sulfonamid
N-[6-Chlor-7-fluor-5-phenyl-1H-indazol-3-yl]-4-(1,1-dimethylethyl)benzolsulfonamid
N-[4-[[(6-Chlor-7-fluor-5-phenyl-1H-indazol-3-yl)amino]sulfonyl]phenyl]acetamid
N-[6-Chlor-7-fluor-5-phenyl-1H-indazol-3-yl]-4-methylbenzolmethansulfonamid
6-Chlor-7-fluor-N-(pentafluorphenyl)-5-phenyl-1H-indazol-3-amin
6-Chlor-7-fluor-N-(3,4-difluorphenyl)-5-phenyl-1H-indazol-3-amin
6-Chlor-7-fluor-5-phenyl-N-(2,3,5,6-tetrafluor-phenyl)-1H-indazol-3-amin
6-Chlor-7-fluor-5-phenyl-N-(2,4,6-trifluorphenyl)-1H-indazol-3-amin
6-chlor-7-fluor-N-(4-fluorphenyl)-5-phenyl-1H-indazol-3-amin
-6-Chlor-7-fluor-N-[3-(trifluomethyl)phenyl]-5-phenyl-1H-indazol-3-amin
6-Chlor-7-fluor-N-[4-(trifluormethyl)phenyl]-5-phenyl-1H-indazol-3-amin
6-Chlor-7-fluor-N-[3-fluor-5-(trifluormethyl)-phenyl]-5-phenyl-1H-indazol-3-amin
6-Chlor-7-fluor-N-(4-nitrophenyl)-5-phenyl-1H-indazol-3-amin
6-chlor-7-fluor-N-(3-nitrophenyl)-5-phenyl-1H-indazol-3-amin
6-chlor-7-fluor-N-(3-methoxyphenyl)-5-phenyl-1H-indazol-3-amin
6-Chlor-7-fluor-N-(4-methoxyphenyl)-5-phenyl-1H-indazol-3-amin
6-Chlor-7-fluor-N,5-diphenyl-1H-indazol-3-amin
6-Chlor-7-fluor-N-(1-pyridinyl)-5-phenyl-1H-indazol-3-amin
6-Chlor-7-fluor-N-(2-pyridinyl)-5-phenyl-1H-indazol-3-amin
N-Butyl-6-chlor-7-fluor-5-phenyl-1H-indazol-3-amin
N-(6-Chlor-7-fluor-5-phenyl-1H-indazol-3-yl)-N'-phenylharnstoff
N-(6-Chlor-7-fluor-5-phenyl-1H-indazol-3-yl)-3-methoxybenzolsulfonamid
Racematen, Enantiomeren und Diastereoisomeren davon und Gemischen davon, Tautomeren davon und pharmazeutisch unbedenkliche Salzen davon.

4. Verbindung nach einem der ansprüche 1 bis 2, **dadurch gekennzeichnet, daß** sie ausgewählt ist unter:
Piperidin-1-carbonsäure(6,7-difluor-5-phenyl-1H-indazol-3-yl)amid
Pyrrolidin-1-carbonsäure(6,7-difluor-5-phenyl-1H-indazol-3-yl)amid
Tautomeren davon sowie pharmazeutisch unbedenklichen Salzen davon.

5. Verbindung, **dadurch** gekenntzeichnet, daß sie ausgewählt ist unter:
1-(6,7-Difluor-5-phenyl-1H-indazol-3-yl)-3-[3-(4-methylpiperazin-1-yl)propyl]harnstoff
Tautomeren davon sowie pharmazeutisch unbedenklichen Salzen davon.

6. Verbindung nach einem der Ansprüche 1 bis 5, die zur Herstellung eines Arzneimittels verwendet wird.

7. Pharmazeutische Zusammensetzung, **dadurch gekennzeichnet, daß** sie in einem pharmazeutisch unbedenklichen Medium eine Verbindung gemäß einem der Ansprüche 1 bis 5 enthält.

8. Arzneimittel nach Anspruch 6, **dadurch gekennzeichnet, daß** es mindestens eine Verbindung gemäß einem der Ansprüche 1 bis 5 enthält, zur therapeutischen Anwendung bei der Behandlung von Erkrankungen, bei denen eine Phosphorylierung des Tau-Proteins beobachtet wird.

9. Arzneimittel nach Anspruch 6, **dadurch gekennzeichnet, daß** es mindestens eine Verbindung gemäß einem der Ansprüche 1 bis 5 enthält, zur therapeutischen Anwendung bei der Behandlung von neurodegenerativen Erkrankungen, Schlaganfällen, Schädel- und Rückenmarkstraumata und peripheren Neuropathien, Obesitas, Stoffwechselerkrankungen, Typ-II-Diabetes, essentieller Hypertonie, atherosklerotischen Herz-Kreislauf-Erkrankungen, Syndrom der polyzystischen Ovarien, Syndrom X, Immundefekt und Krebs.

10. Medikament nach Anspruch 9, **dadurch gekennzeichnet, daß** es sich bei der neurodegenerativen Erkrankung um Alzheimer-Krankheit, Parkinson-Krankheit, frontoparietale Demenz, kortikobasale Degeneration oder Pick-Krankheit handelt.

11. Verfahren zur Herstellung der Verbindungen der Formel (I) gemäß Anspruch 1, für die R3 für (1-6C)-Alkyl, Aryl-(1-6C)-alkyl, Heteroaryl-(1-6C)-alkyl, Heterocycloalkyl, Cycloalkyl oder Polycycloalkyl steht, wobei diese Reste gegebenenfalls durch i oder mehrere unter Halogen, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2-, C(S)NR1R2, NHC(S)R1, -O-SO₂R1, -SO₂-O-R1, Aryl, heteroaryl, Formyl, Oxo, Trifluormethyl, Trifluormethylsulfanyl, Trifluormethoxy und (1-6C)-Alkyl ausgewählte Substituenten substituiert sind; und R1 und R2 unabhängig voneinander für Wasserstoff, (1-6C)-Alkyl, Aryl, Alkenyl, Alkinyl oder Heteroaryl, die selbst gegebenenfalls durch 1 oder mehrere unter Halogen, (1-6C)-Alkyl, (1-6C)-Alkoxy, CN, NO₂, NH₂, OH, COOH, COO-Alkyl, CONH₂, Formyl, Oxo, Trifluormethyl und Trifluormethoxy ausgewählte Substituenten substituiert sind, stehen; aus einem Derivat der Formel (I), worin R3 für H steht, einem R1CHO-Derivat und Natriumtriacetoxyborhydrid in Dichlormethan, und gegebenenfalls Umwandlung des erhaltenen Produkts in ein pharmazeutisch unbedenkliches Salz.

12. Verfahren zur Herstellung der Verwindungen der Formel (I) gemäß Anspruch 1, für die R3 für CONR1R2 oder CSNR1R2 steht und R1 und R2 unabhängig voneinander für Wasserstoff, (1-6C)-Alkyl, Aryl, Alkenyl, Alkinyl oder Heteroaryl, die selbst gegebenenfalls durch 1 oder mehrere unter Halogen, (1-6C)-Alkyl, (1-6C)-Alkoxy, CN, NO₂, NH₂, OH, COOH, COO-Alkyl, CONHₐ, Formyl, Oxo, Trifluorethyl und Trifluormethoxy ausgewählte Substituenten substituiert sind, stehen; aus OCNR1 und einem Derivat der Formel (I), worin R3 für H steht, in Tetrahydrofuran, und gegebenenfalls Umwandlung des erhaltenen Produkts in ein pharmazeutisch unbedenkliches Salz.

13. Verfahren zur Herstellung der Verbindungen der Formel (I) gemäß Anspruch 1, für die R3 für SO₂R1 steht und R1 für wasserstoff, (1-6C)-Alkyl, Aryl, Alkenyl, Alkinyl oder Heteroaryl, die selbst gegebenenfalls durch 1 oder mehrere unter Halogen, (1-6C)-Alkyl, (1-6C)-Alkoxy, CN, NO₂, NH₂, OH, COOH, COO-Alkyl, CONH₂, Formyl, Oxo, Trifluormethyl und Trifluormethoxy ausgewählte Substituenten substituiert sind, steht; aus einem Sulfonylchlorid R1SO₂C1 und einem Derivat oder Formel (I), worin R3 für H steht, in Dichlormethan in Gegenwart einer Base, und gegebenenfalls Umwandlung des erhaltenen Produkts in ein pharmazeutisch unbedenkliches Salz.

14. Als Zwischenprodukte
N-(6,7-Diflunr-1H-indazol-3-yl)butanamid
N-[6,7-Difluor-1-[[2-(trimethylsilyl)ethoxy]-methyl]-1H-indazol-3-yl]butanamid
N-[5-Brom-6,7-difluoro-1-[[2-(trimethylsilyl)-ethoxy]methyl]-1H-indazol-3-yl]butanamid
N-[6,7-Difluor-5-phenyl-1-[[2-(trimethylsilyl)-ethoxy]methyl]-1H-indazol-3-yl]butanamid
6,7-Difluor-5-phenyl-1-[[2-(trimethylsilyl)-ethoxy]methyl]-1H-indazol-3-amin.
